# EUROPEAN PATENT SPECIFICATION

(11) **EP 3 989 962 B1**
(45) Date of publication and mention of the grant of the patent: **27.08.2025**
(21) Application number: 20736444.9
(22) Date of filing: 26.06.2020
(51) Int. Cl.: A61K 31/198, A61K 31/445, A61P 3/00, A61P 25/28

(54) **COMBINATION THERAPY WITH ACETYL-LEUCINE AND MIGLUSTAT FOR TREATING A LYSOSOMAL STORAGE DISEASE**
KOMBINATIONSTHERAPIE MIT ACETYL-LEUCIN UND MIGLUSTAT ZUR BEHANDLUNG VON LYSOSOMALE SPEICHERKRANKHEITEN
POLYTHÉRAPIE COMPRENANT DE L'ACÉTYL-LEUCINE ET DU MIGLUSTAT POUR LE TRAITEMENT D'UNE MALADIE LYSOSOMALE

(30) Priority: 28.06.2019 US 201962868383 P; 05.11.2019 US 201962931003 P
(43) Date of publication of application: 04.05.2022
(73) Proprietor: IntraBio Ltd, London NW3 6BP (GB)
(72) Inventor: FACTOR, Mallory, Mayfair W1J 5LF (GB); STRUPP, Michael, 81377 Munich (DE)
(74) Representative: Maiwald GmbH
(86) International application number: PCT/IB2020/056096
(87) International publication number: WO 2020/261230

(56) References cited:
- WO-A1-2018/029657
- TATIANA BREMOVA ET AL: "Acetyl-DL-leucine in Niemann-Pick type C: A case series", NEUROLOGY, vol. 85, no. 16, 20 October 2015 (2015-10-20), US, pages 1368 - 1375, XP055390576, ISSN: 0028-3878, DOI: 10.1212/WNL.0000000000002041
- MARIO CORTINA-BORJA ET AL: "Annual severity increment score as a tool for stratifying patients with Niemann-Pick disease type C and for recruitment to clinical trials", ORPHANET JOURNAL OF RARE DISEASES, vol. 13, no. 1, 16 August 2018 (2018-08-16), XP055730680, DOI: 10.1186/s13023-018-0880-9
- PINEDA M ET AL: "Miglustat in patients with Niemann-Pick disease Type C (NP-C): A multicenter observational retrospective cohort study", MOLECULAR GENETICS AND METABOLISM, ACADEMIC PRESS, AMSTERDAM, NL, vol. 98, no. 3, 1 November 2009 (2009-11-01), pages 243 - 249, XP026683227, ISSN: 1096-7192, [retrieved on 20090804], DOI: 10.1016/J.YMGME.2009.07.003
- PATTERSON ET AL: "Miglustat for treatment of Niemann-Pick C disease: a randomised controlled study", LANCET NEUROLOGY, LANCET PUBLISHING GROUP, LONDON, GB, vol. 6, no. 9, 14 August 2007 (2007-08-14), pages 765 - 772, XP022200287, ISSN: 1474-4422, DOI: 10.1016/S1474-4422(07)70194-1
- HAMMOND NATALIE ET AL: "The complexity of a monogenic neurodegenerative disease: More than two decades of therapeutic driven research into Niemann-Pick type C disease", BIOCHIMICA ET BIOPHYSICA ACTA (BBA) - MOLECULAR AND CELL BIOLOGY OF LIPIDS, vol. 1864, no. 8, 17 April 2019 (2019-04-17), pages 1109 - 1123, XP085704057, ISSN: 1388-1981, DOI: 10.1016/J.BBALIP.2019.04.002
- FRANCES M. PLATT: "Emptying the stores: lysosomal diseases and therapeutic strategies", NATURE REVIEWS DRUG DISCOVERY, vol. 17, no. 2, 17 November 2017 (2017-11-17), GB, pages 133 - 150, XP055730691, ISSN: 1474-1776, DOI: 10.1038/nrd.2017.214
- BREMOVA-ERTL T ET AL: "EPR1131: Acetyl-Leucine slows disease progression in lysosomal storage disorders", EUROPEAN JOURNAL OF NEUROLOGY : THE OFFICIAL JOURNAL OF THE EUROPEAN FEDERATION OF NEUROLOGICAL SOCIETIES; ABSTRACTS OF THE 6TH CONGRESS OF THE EUROPEAN ACADEMY OF NEUROLOGY, VIRTUAL 2020, OXFORD [U.A.] : WILEY-BLACKWELL, GB, vol. 27, no. Supplement 1, 1 May 2020 (2020-05-01), pages 181, XP009522922, ISSN: 1468-1331, [retrieved on 20200522], DOI: 10.1111/ENE.14307

## Description

### BACKGROUND OF THE INVENTION

### Field of the Invention

The present disclosure provides a combination of acetyl-leucine and miglustat for treating lysosomal storage disorders (LSDs) in a subject, wherein the subject is naive to treatment with miglustat.

### Background

Lysosomal storage disorders (LSDs) are a group of inherited metabolic diseases caused by defects in lysosomal homeostasis. LSDs encompass over 70 diseases, with a collective clinical frequency of 1:5000 live births. These diseases can be classified into two main groups: primary storage disorders resulting from a direct deficiency in degradation pathways (typically lysosomal enzyme deficiency disorders), and secondary storage disorders which are caused by malfunctioning downstream lysosomal proteins or processes that impact the lysosome (e.g., defects in trafficking pathways).

The pathology of LSDs affects many of the body's systems, but most commonly the nervous system. Progressive neurodegeneration resulting in physical disability and mental deterioration are common symptoms. Such disorders are generally severely progressive and unremitting. They tend to present in the first few years of life and the severe progression results in frequent hospitalization. If left untreated, patients often die in their mid-teens. Adult-onset patients have also been described.

Current therapeutic approaches to treat LSDs are limited. Miglustat is regarded as the first-line therapy for treating Niemann-Pick type C disease (NPC), Patterson et al., Lancet Neurol 6:765-772 (2007); Hammond et al., Biochim Biophys Acta Mol Cell Biol Lipids 1864(8):1109-1123 (2019); Platt: Nat Rev Drug Discov; 17(2): 133-150 (2018), but this drug causes unwanted weight loss and gastrointestinal disturbances in the treatment subjects. Champion et al., J Inherit Metab Dis 33 Suppl 3:S379-83 (2010). Acetyl-DL-leucine can also be used to treat NPC as a single agent, Bremova-Ertl et al., Eur J Neurol. Suppl 1:1-1228. vol. 27, (2020), and has been administered as an add-on therapy in NPC subjects receiving miglustat as a first-line therapy. Bremova et al., Neurology 85:1368-1375 (2015); Cortina-Borja et al., Orphanet Journal of Rare Diseases 13:143 (2018); WO 2018/029657. These add-on studies did not include NPC subjects naive to treatment with miglustat and were not designed to measure the combined therapeutic effects of acetyl-DL-leucine and miglustat. There is a need for improved treatments of LSDs with enhanced efficacy and an improved safety profile for first-line therapy.

### BRIEF SUMMARY OF THE INVENTION

The subject matter of the invention is as defined in the appended claims.

In all instances, disclosures relating to methods of treatment comprising administering acetyl-leucine and miglustat are to be understood as purpose-limited product claims, i.e. acetyl-leucine and miglustat for use in such methods of treatment.

Applicant has unexpectedly discovered that acetyl-leucine synergizes with miglustat to treat NPC, e.g., slow disease progression. Also, without wishing to be bound by any particular theory, it is believed that the combination of acetyl-leucine and miglustat will lessen the toxicity, e.g., weight loss and/or appetite suppression, typically associated with the administration of miglustat.

In one aspect, the present disclosure provides acetyl-leucine in combination with miglustat for use in the treatment of a LSD in a subject, wherein the subject is naive to treatment with miglustat.

In another aspect, the present disclosure provides a method of treating a LSD in a subject in need thereof, the method comprising administering a combination of (i) a therapeutically effective amount of acetyl-leucine; and (ii) a therapeutically effective amount of miglustat, to the subject, wherein the subject is naive to treatment with miglustat.

In another aspect, the present disclosure provides the combination of acetyl-leucine and miglustat for first-first line therapy to treat a LSD.

In another aspect, the present disclosure provides a kit comprising acetyl-leucine and miglustat for use in a method of treating a lysosomal storage disease in a subject, wherein subject is naive to treatment with miglustat.

It is to be understood that both the foregoing summary and the following detailed description are exemplary and explanatory only, and are not restrictive of the invention as claimed.

### DETAILED DESCRIPTION OF DRAWINGS

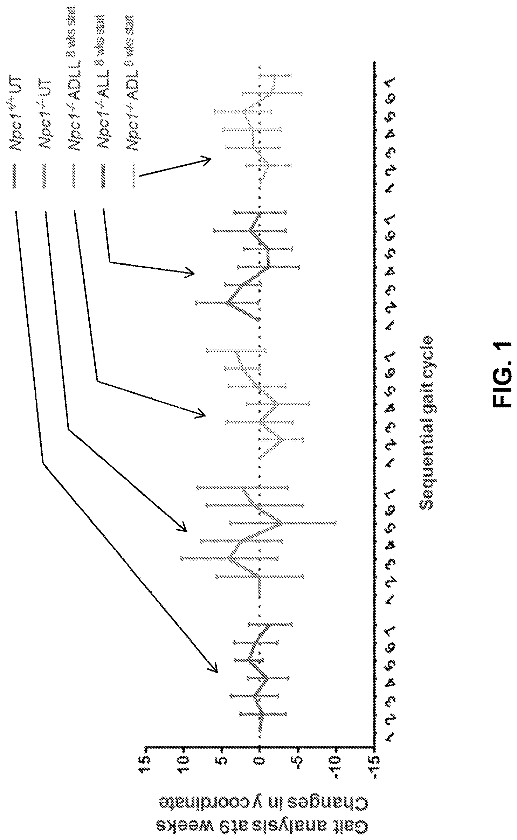

Fig. 1 is a line graph showing the late stage Y coordinate displacement of each consecutive foot from a straight-line trajectory (Mean ± SD, n = 6) for untreated wild type (*Npc1*^{*+*/}*⁺UT*) and NPC1 (*Npc1*^{*-*/*-*} UT) mice, and ADLL (*Npc1*^{*-*/*-*} ADLL), ALL (*Npc1*^{*-*/*-*} ALL), and ADL (*Npc1*^{*-*/*-*} ADL) treated NPC1 mice (minimum 5 animals, maximum 7 animals for each group).

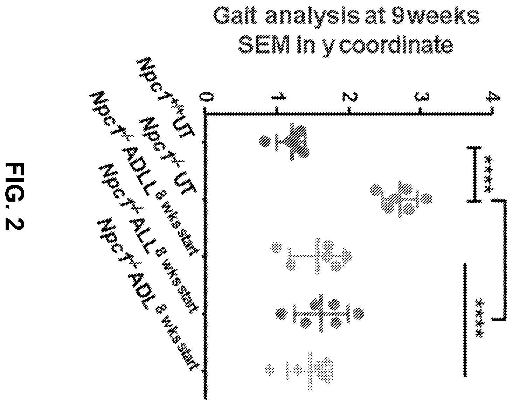

Fig. 2 is a column scatter graph showing the late stage SEMs of the Y coordinate changes (mean ± SD, n = 6; *p* <0.0001, One-way ANOVA)) for untreated wild type (*Npc1*^{*+*/}*⁺UT*) and NPC1 (*Npc1*^{*-*/*-*} UT) mice, and ADLL (*Npc1*^{*-*/*-*} ADLL), ALL (*Npc1*^{*-*/*-*} ALL), and ADL (*Npc1*^{*-*/*-*} ADL) treated NPC1 mice.

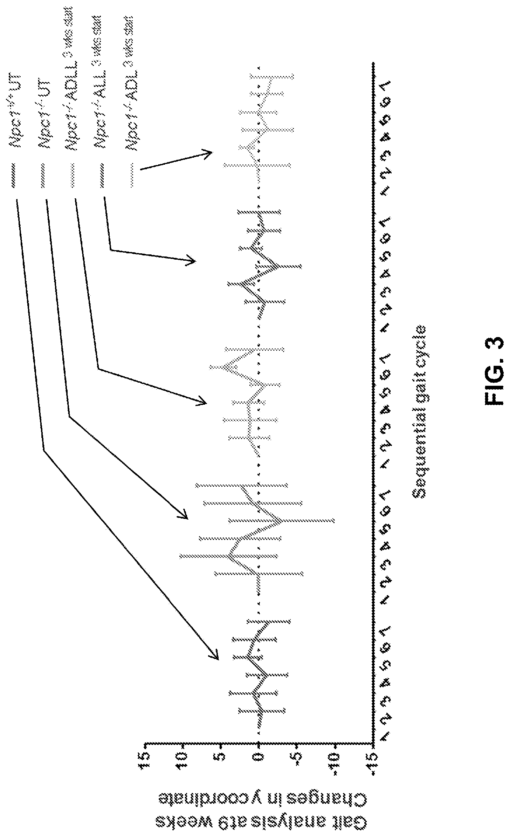

Fig. 3 is a line graph showing the early stage Y coordinate displacement of each consecutive foot from a straight-line trajectory (mean ± SD, n = 6)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-* /-} ALL), and ADL (*Npc1*^{*-*/*-*} ADL) treated NPC1 mice.

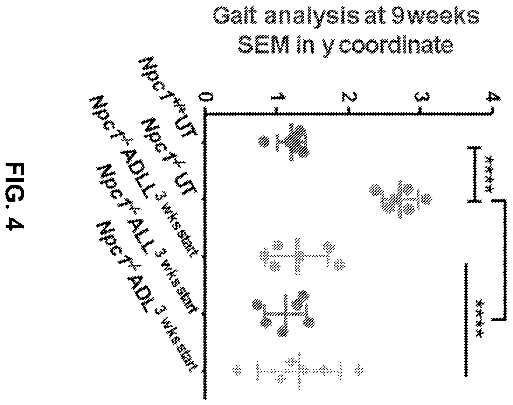

Fig. 4 is a column scatter graph showing the early stage SEMs of the Y coordinate changes (mean ± SD, n = 6; *p* <0.0001, One-way ANOVA)) for untreated wild type (*Npc1*^{*+*/}*⁺UT*) and NPC1 (*Npc1*^{*-*/*-*} UT) mice, and ADLL (*Npc1*^{*-*/*-*} ADLL), ALL (*Npc1*^{*-*/*-*} ALL), and ADL (*Npc1*^{*-*/*-*} ADL) treated NPC1 mice.

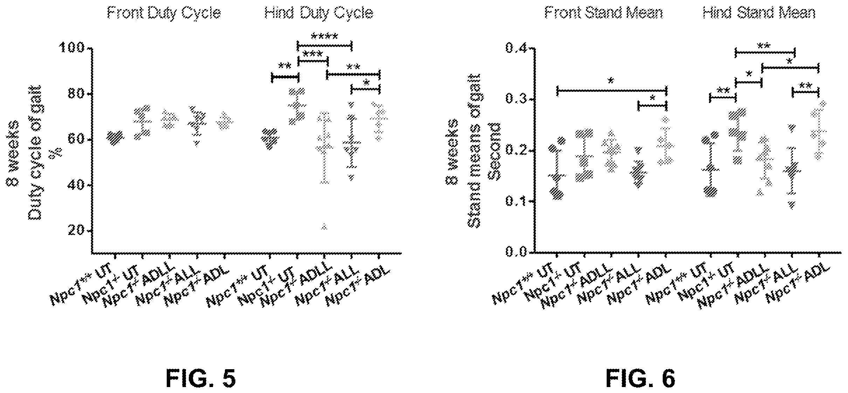

Fig. 5 is a column scatter graph showing the early stage duty cycle with front and hind duty cycle measurements (mean ± SD, ***p* <0.032, ****p* <0.0005, *****p <0.0001* (2-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), and ADL (*Npc1*^{*-*/*-*} ADL) treated NPC1 mice (minimum 5 animals, maximum 7 animals for each group).
Fig. 6 is a column scatter graph showing the early stage front and hind stand mean measurements (mean ± SD, **p* <0.026, ***p* <0.003 (2-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-* /-} ALL), and ADL (*Npc1*^{*-*/*-*} ADL) treated NPC1 mice (minimum 5 animals, maximum 7 animals for each group).

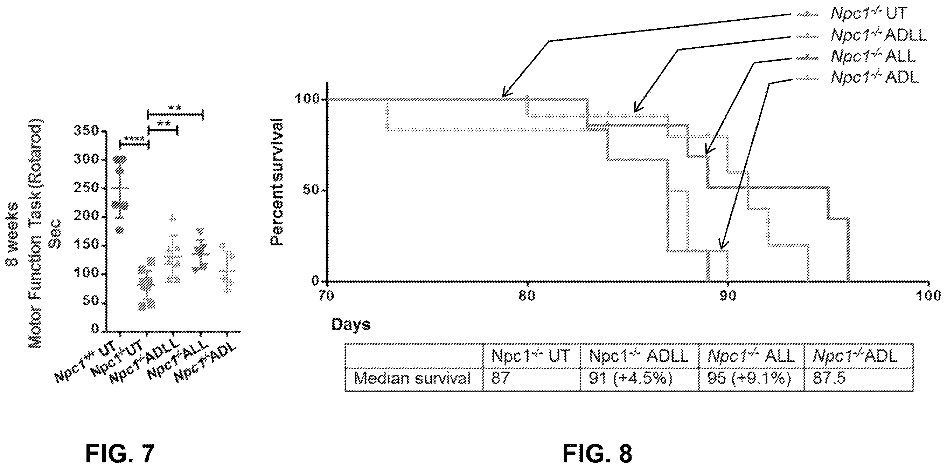

Fig. 7 is a column scatter graph showing the early stage motor performance measurements (mean ± SD, ***p* <0.009, *****p* <0.0001 (One-way ANOVA)) for untreated wild type (*Npc1*^{*+*/*+*}UT) and NPC1 (*Npc1*^{*-*/*-*} UT) mice, and ADLL *(Npc1*^{*-*/*-*} ADLL), ALL (*Npc1*^{*-*/*-*} ALL), and ADL (*Npc1*^{*-*/*-*} ADL) treated NPC1 mice (minimum 5 animals, maximum 7 animals for each group).
Fig. 8 is a line graph showing the life expectancy percentages (mean ± SD, **p* <0.046 (Gehan-Breslow-Wilcoxon test) n = 6) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), and ADL (*Npc1*^{*-*/*-*} ADL) treated NPC1 mice.

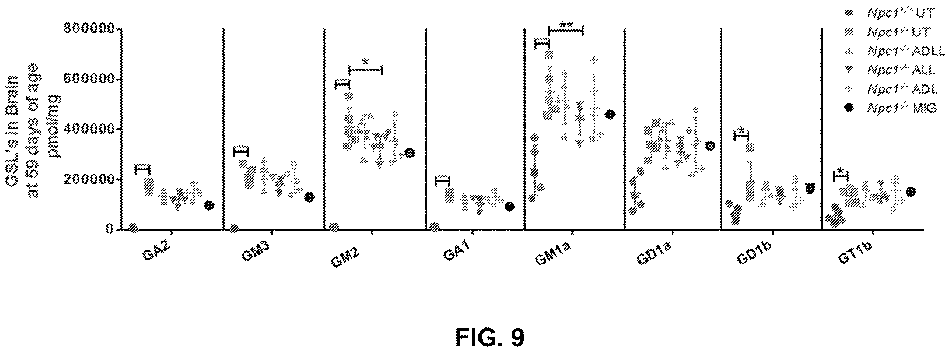

Fig. 9 is a column scatter graph showing the glycosphingolipid (GSL) profiles in brain (mean ± SD, **p* <0.024, ***p* <0.0019, ****p* <0.0005, *****p* <0.0001 (2-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.

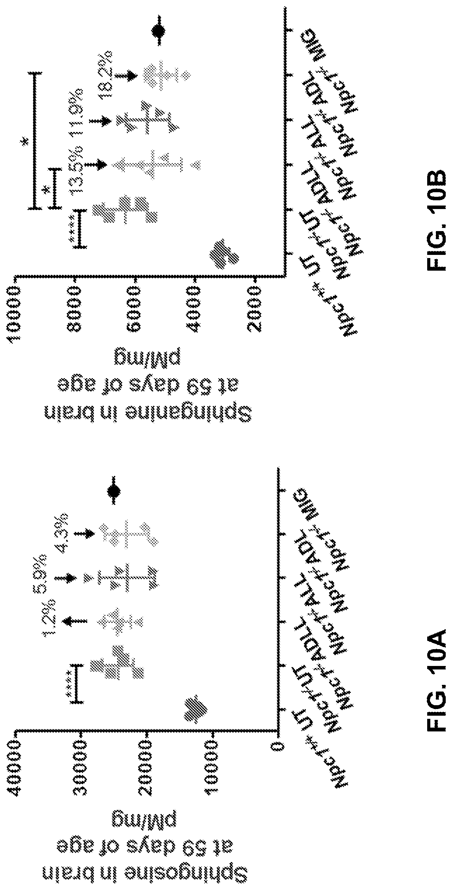

Fig. 10A is a column scatter graph showing the sphingosine levels in brain (mean ± SD, *****p* <0.0001 (One-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.
Fig. 10B is a column scatter graph showing the sphinganine levels in brain (mean ± SD, **p* <0.046, *****p* <0.0001 (One-way ANOVA)) for untreated wild type (*Npc1*^{*+*/*+*} UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.

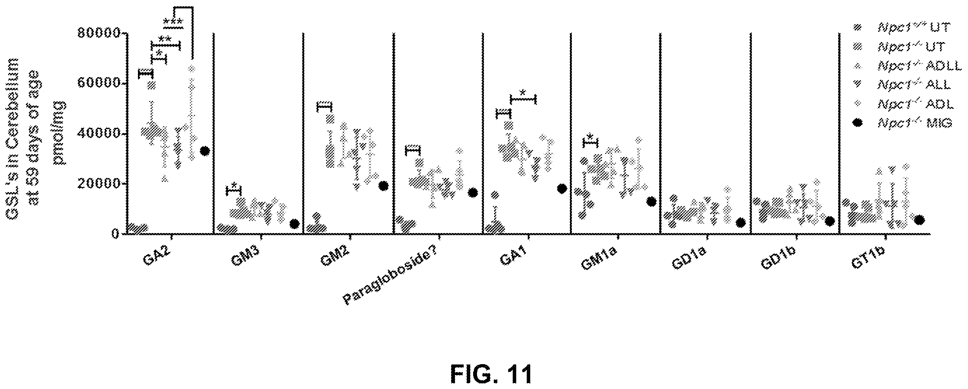

Fig. 11 is a column scatter graph showing the GSL profiles in the cerebellum (mean ± SD, **p* <0.026, ***p* <0.005, ****p* <0.0009, *****p* <0.0001 (2-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.

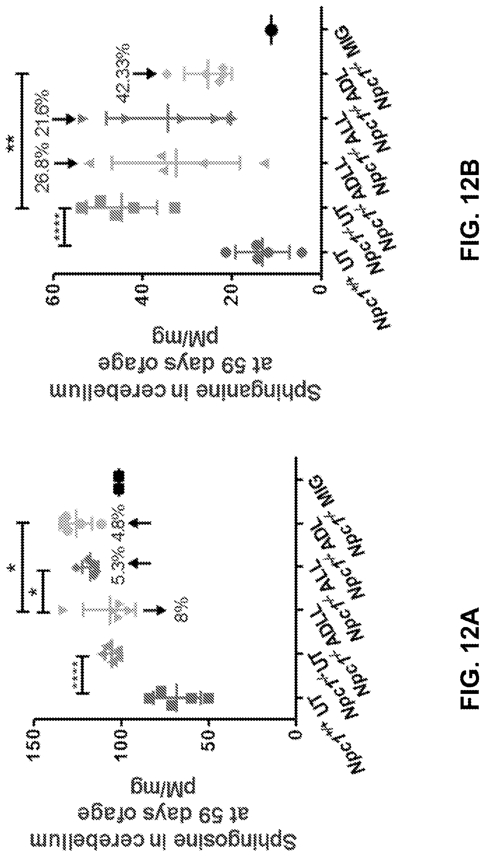

Fig. 12A is a column scatter graph showing the sphingosine levels in the cerebellum (mean ± SD, **p* <0.046 *****p* <0.0001 (One-way ANOVA)) for untreated wild type (*Npc1*^{*+*/}*⁺UT*) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.
Fig. 12B is a column scatter graph showing the sphinganine levels in brain without the cerebellum (mean ± SD, ***p* <0.075, *****p* <0.0001(One-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.

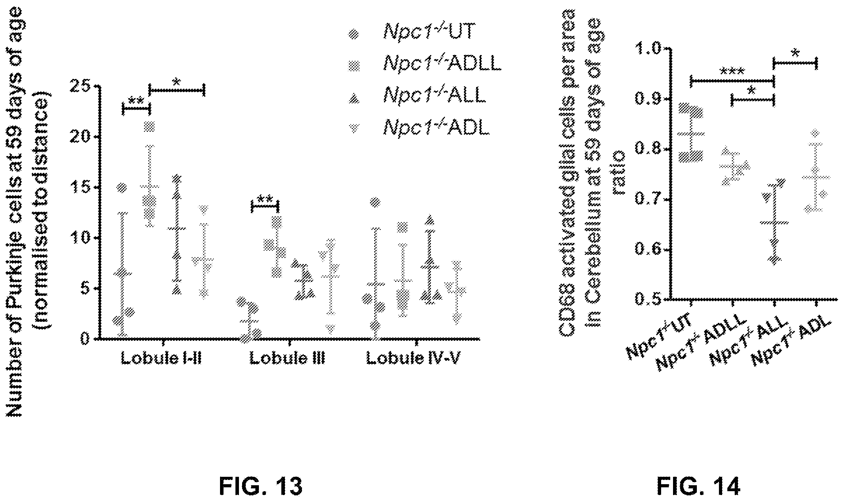

Fig. 13 is a column scatter graph showing the purkinje cell density (mean ± SD, **p* <0.011, ***p* <0.028 (One-way ANOVA)) for NPC1 untreated (*Npc1*^{*-*/*-*} UT), and ADLL (*Npc1*^{*-*/*-*} ADLL), ALL (*Npc1*^{*-*/*-*} ALL), and ADL (*Npc1*^{*-*/*-*} ADL) treated mice at 59 days of age. For all AL treatments n = 5 animals per group.
Fig. 14 is a column scatter graph showing the CD68 cell density (mean ± SD, **p* <0.046, ****p* <0.0009 (One-way ANOVA)) for NPC1 untreated (*Npc1* UT), and ADLL (*Npc1*^{*-*/*-*} ADLL), ALL (*Npc1*^{*-*/*-*} ALL), and ADL (*Npc1*^{*-*/*-*} ADL) treated mice at 59 days of age. For all AL treatments n = 5 animals per group.

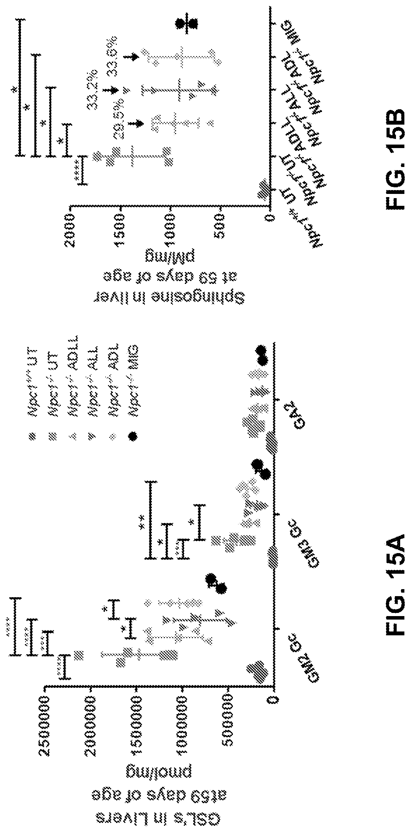

Fig. 15A is a column scatter graph showing the GSL profile in liver (mean ± SD **p* <0.038, ****p* <0.0002, *****p* <0.0001 (2-way ANOVA)) for untreated wild type (*Npc1*^{*+*/}*⁺UT*) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.
Fig. 15B is a column scatter graph showing the sphingosine levels in liver (mean ± SD. **p* <0.0393, *****p* <0.0001(One-way ANOVA)) for untreated wild type (*Npc1*^{*+*/*+*} UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.

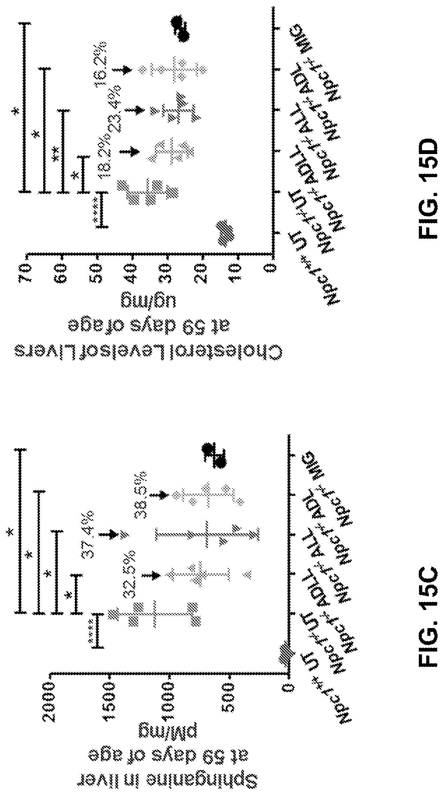

Fig. 15C is a column scatter graph showing the sphinganine levels in liver (mean ± SD, **p* <0.04, *****p* <0.0001(One-way ANOVA)) for untreated wild type (*Npc1*^{*+*/*+*} UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.
Fig. 15D is a column scatter graph showing the liver cholesterol (mean ± SD, **p* <0.034, ***p =* 0.0091, *****p* <0.0001 (One-way ANOVA)) for untreated wild type (*Npc1*^{*+*/}*⁺UT*) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL), and miglustat (*Npc1*^{*-*/*-*} MIG) treated NPC1 mice at 59 days of age. For all AL treatments n = 5 animals per group, and for miglustat (positive control) n = 2 or n = 1.

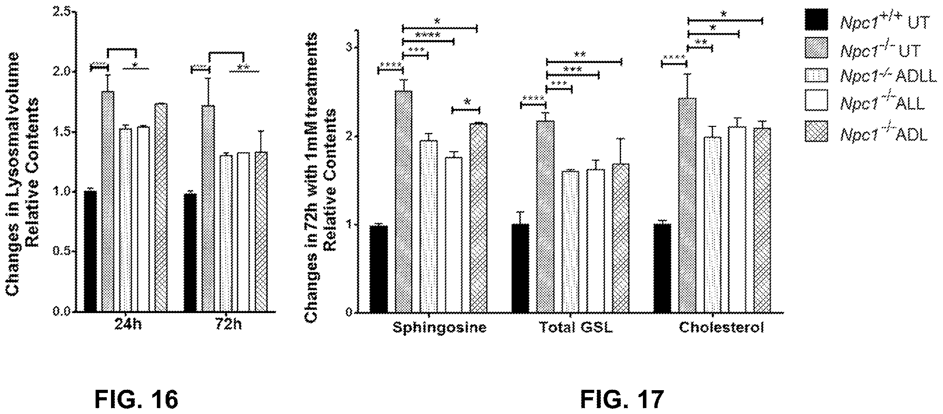

Fig. 16 is a bar graph showing the Lysotracker Green relative to *Npc1*^{*+*/*+*} with 24 and 72 h 1 mM AL treatment (mean ± SD, **p* <0.018, ***p* <0.005, *****p* <0.0001 (2-way ANOVA)) in mice at 59 days of age. For all AL treatments n = 5 animals per group.
Fig. 17 is a bar graph showing the sphingosine, total GSL and free cholesterol levels (72 h) relative to *Npc1*^{*+*/*+*} with 72 h 1 mM AL treatment (mean ± SD, **p* <0.027, ***p* <0.0044, ***p <0.001 *****p* <0.0001 (2-way ANOVA)) in mice at 59 days of age. For all AL treatments n = 5 animals per group.

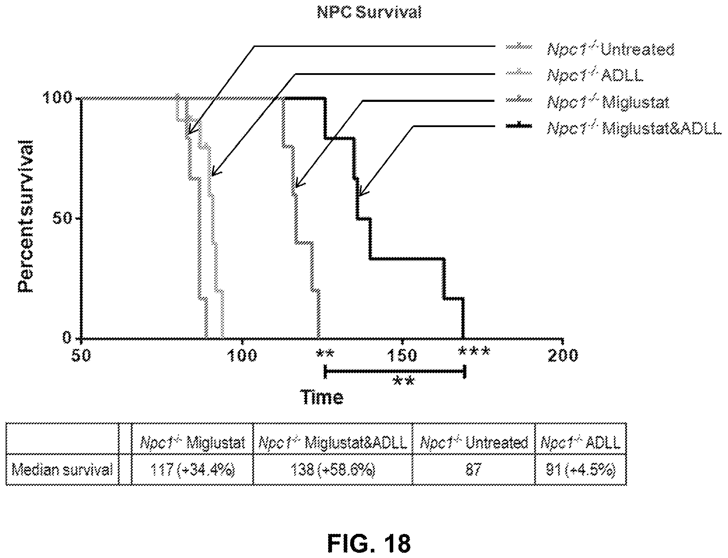

Fig. 18 is a line graph showing the survival curves (mean ± SD, **p* = 0.0270, ***p* <0.0035, ****p* = 0.0007 (Gehan-Breslow-Wilcoxon test)) and an analysis table for NPC1 untreated (*Npc1*^{*-*/*-*} UT), ADLL (*Npc1*^{*-*/*-*} ADLL), miglustat (*Npc1*^{*-*/*-*} MIG), and combination (*Npc1*^{*-*/*-*} miglustat & ADLL) treated mice with a minimum of 5 animals for each group.

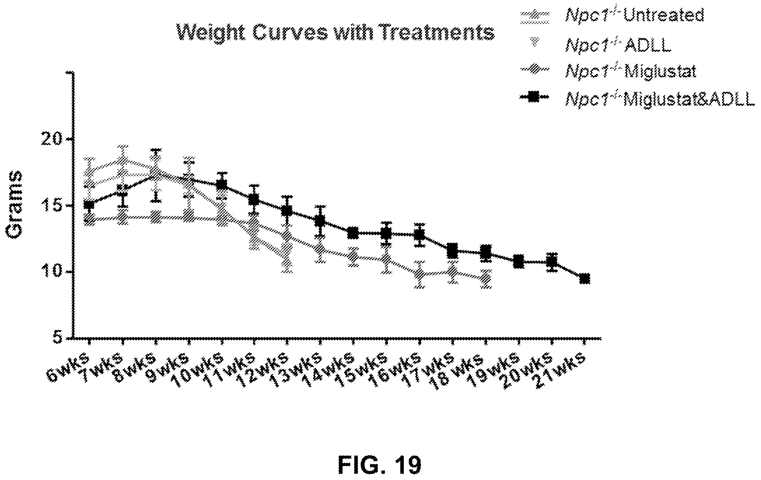

Fig. 19 is a line graph showing the body weight curves over time (mean ± SD) for NPC1 untreated (*Npc1*^{*-*/*-*} UT), ADLL (*Npc1*^{*-*/*-*} ADLL), miglustat (*Npc1*^{*-*/*-*} MIG), and combination (*Npc1*^{*-*/*-*} miglustat & ADLL) treated mice with a minimum of 5 animals for each group.

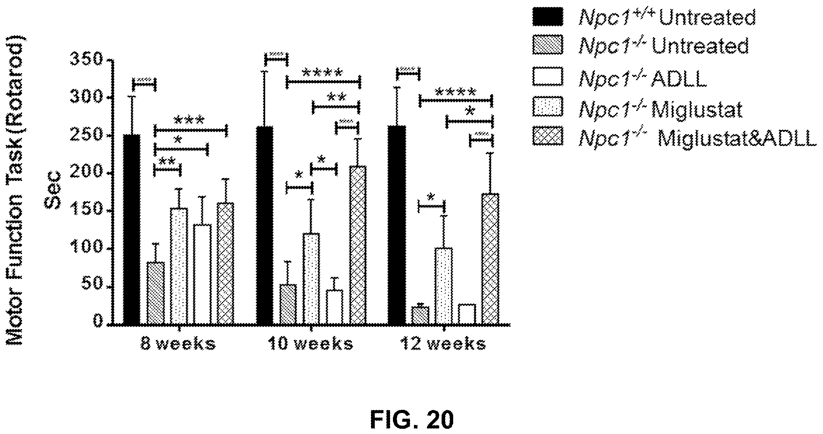

Fig. 20 is a bar graph showing the motor performance measurements at 8, 10, and 12 weeks of age (mean ± SD, **p* <0.043, ***p* <0.0036, *****p <* 0.0001 (2-way ANOVA)) for untreated wild type (*Npc1*^{*+*/*+*}UT) and NPC1 (*Npc1*^{*-*/*-*} UT) mice, and ADLL (*Npc1*^{*-*/*-*} ADLL), miglustat (*Npc1*^{*-*/*-*} MIG), and combination (*Npc1*^{*-*/*-*} miglustat & ADLL) treated NPC1 mice with a minimum of 5 animals for each group.

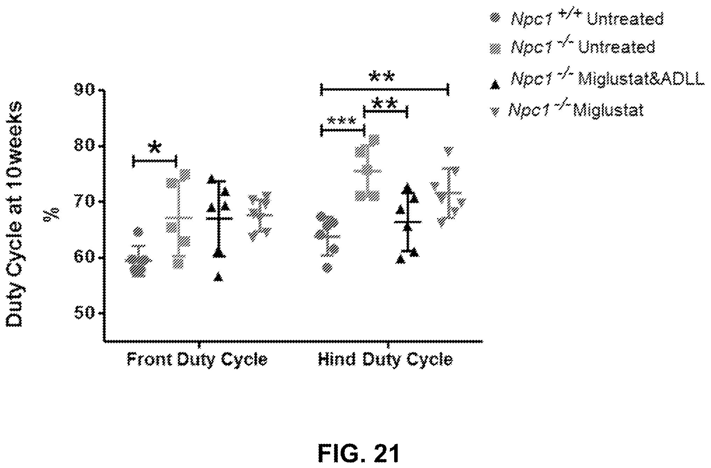

Fig. 21 is a column scatter graph showing the duty cycle measurements of the front and hind paws at 10 weeks of age (mean ± SD, **p =* 0.011, ***p* <0.0053, ****p* 0.0002 (2-way ANOVA)) for untreated wild type (*Npc1*^{*+*/}*⁺UT*) and NPC1 (*Npc1*^{*-*/*-*} UT) mice, and miglustat (*Npc1*^{*-*/*-*} MIG) and combination (*Npc1*^{*-*/*-*} miglustat & ADLL) treated NPC1 mice with a minimum of 5 animals for each group.

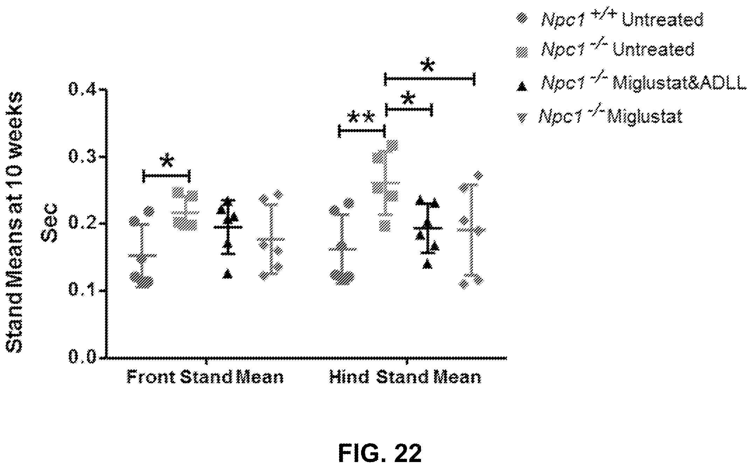

Fig. 22 is a column scatter graph showing the stand mean measurements of the front and hind paws at 10 weeks of age, (mean ± SD, **p* <0.033, ***p* <0.0016 (2-way ANOVA)) for untreated wild type (*Npc1*^{*+*/*+*}UT) and NPC1 (*Npc1*^{*-*/*-*} UT) mice, and miglustat (*Npc1*^{*-*/*-*} MIG) and combination (*Npc1*^{*-*/*-*} miglustat & ADLL) treated NPC1 mice with a minimum of 5 animals for each group.

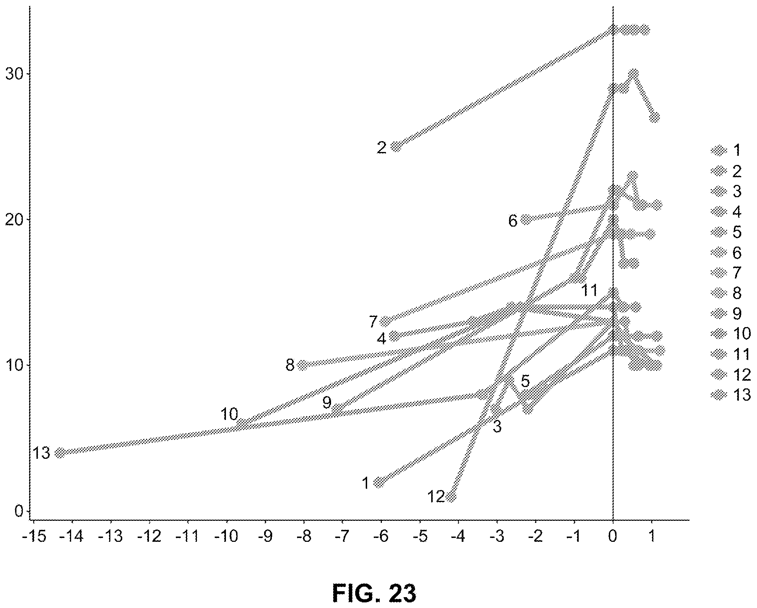

Fig. 23 is a line graph showing the clinical data from 13 adult NPC patients (for demographics see EXAMPLE 13 and General Methods) showing disease progression (clinical severity scale on the Y-axis) over time (Years, X- axis) prior to the initiation of treatment with ADLL (indicated by the vertical line).

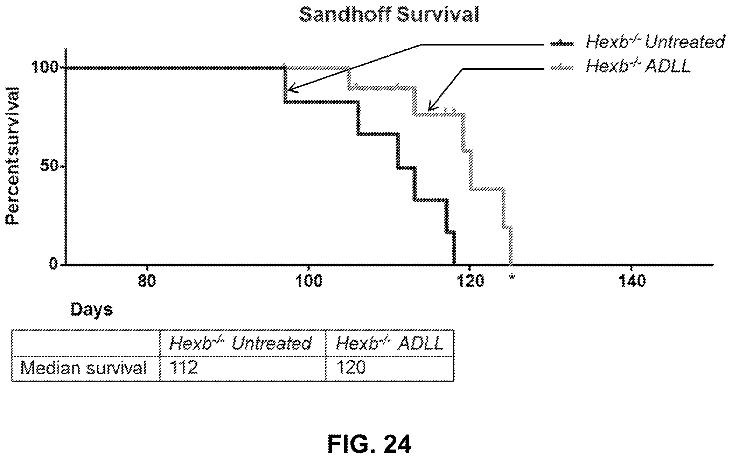

Fig. 24 is a line graph showing the changes in life expectancy ((%), mean ± SD, **p* = 0.0157 (Gehan-Breslow-Wilcoxon test) n = 5) and an analysis table in Sandhoff untreated (*Hexb*^{*-*/*-*} UT) and ADLL (*Hexb*^{*-*/}*⁻ADLL*) treated mice.

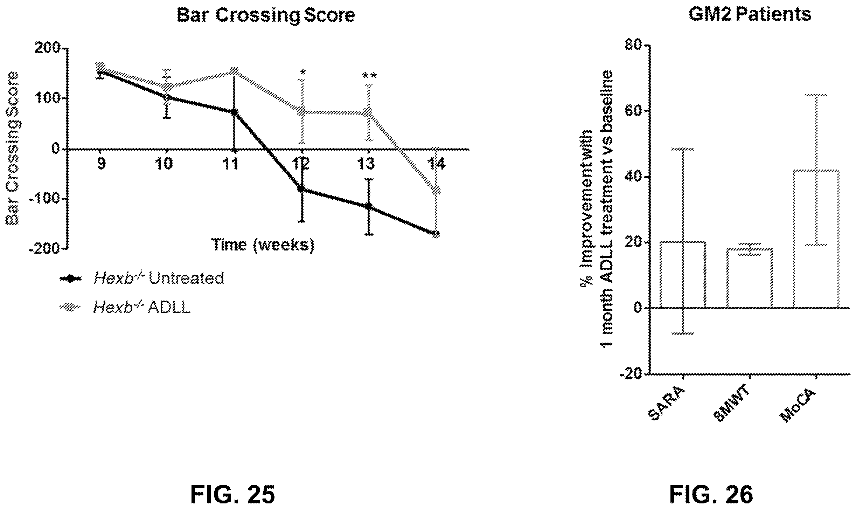

Fig. 25 is a line graph showing the bar crossing score (**p* = 0.0343, ***p* = 0.0058, Mean ± SD, 2-way ANOVA) in Sandhoff untreated (*Hexb*^{*-*/*-*} UT) and ADLL (*Hexb*^{*-*/*-*} ADLL) treated mice.
Fig. 26 is a bar graph showing the percent improvement in clinical scores in three patients with GM2 gangliosidosis at baseline and after one month on medication ADLL. The Scale for Assessment and Rating of Ataxia (SARA) changed by 20.3% and the 8-M-Walking-Test (8MWT) changed by 17.8%. Cognitive function, as assessed by Montreal Cognitive Assessment (MoCA) changed by 42.0%.

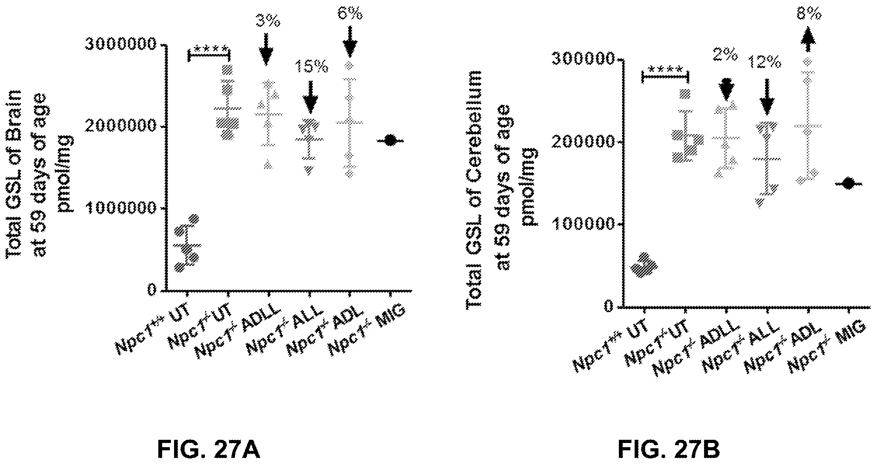

Fig. 27A is a column scatter graph showing the total GSL levels in the brain (mean ± SD, **p* <0.034, ****p =* 0.0004, (2-way ANOVA)) of untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL) (5 animals per group), and miglustat (*Npc1*^{*-*/*-*} MIG) (1 or 2 animals per group) treated NPC1 mice at 59 days of age.
Fig. 27B is a column scatter graph showing the total GSL levels in the cerebellum (mean ± SD, (2-way ANOVA)) of untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), and ADL (*Npc1*^{*-*/*-*} ADL) (5 animals per group), and miglustat (*Npc1* MIG) (1 or 2 animals per group) treated NPC1 mice at 59 days of age.

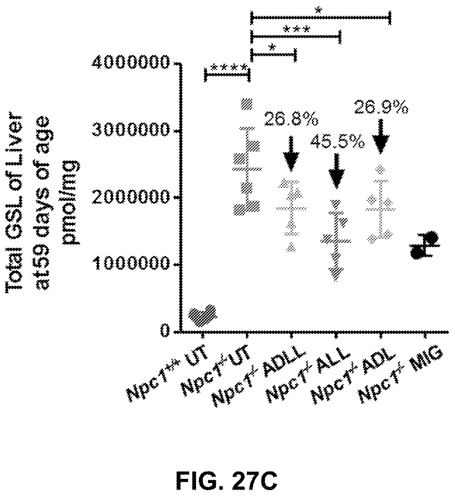

Fig. 27C is a column scatter graph showing the total GSL levels in the liver (mean ± SD, (2-way ANOVA)) of untreated wild type (*Npc1*^{*+*/*+*}UT) and NPC1 (*Npc1*^{*-*/*-*} UT) mice, and ADLL (*Npc1*^{*-*/*-*} ADLL), ALL (*Npc1*^{*-*/*-*} ALL), ADL (*Npc1*^{*-*/*-*} ADL) (5 animals per group), and miglustat (*Npc1*^{*-*/*-*} MIG) (1 or 2 animals per group) treated NPC1 mice at 59 days of age.

### DETAILED DESCRIPTION OF THE INVENTION

In one embodiment, the present disclosure provides acetyl-leucine in combination with miglustat for use in the treatment of a lysosomal storage disease (LSD) in a subject in need thereof, wherein the subject is naive to treatment with miglustat

### Definitions

As used herein, the singular forms "a," "an," and "the" include plural reference.

As used herein, the terms "approximately" and "about" should be generally understood to encompass ± 20% of a specified amount, frequency or value. Numerical quantities given herein are approximate unless stated otherwise, meaning that term "about" or "approximately" can be inferred when not expressly stated.

The terms "administer," "administration," or "administering" as used herein refer to (1) providing, giving, dosing and/or prescribing by either a health practitioner or his authorized agent or under his direction, the combination of acetyl-leucine and miglustat, and (2) putting into, taking or consuming by the subject or person himself or herself, acetyl-leucine and miglustat. Any reference to acetyl-leucine and miglustat include pharmaceutically acceptable salts of the same, even if not expressly stated.

A "pharmaceutically acceptable salt" as referred to herein, is any salt preparation that is appropriate for use in a pharmaceutical application. Pharmaceutically acceptable salts include, but are not limited to, amine salts, such as N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine, tris(hydroxymethyl)aminomethane and the like; alkali metal salts, such as lithium, potassium, sodium and the like; alkali earth metal salts, such as barium, calcium, magnesium and the like; transition metal salts, such as zinc, aluminum and the like; other metal salts, such as sodium hydrogen phosphate, disodium phosphate and the like; mineral acids, such as hydrochlorides, sulfates and the like; and salts of organic acids, such as acetates, lactates, malates, tartrates, citrates, ascorbates, succinates, butyrates, valerates, fumarates and the like.

Acetyl-leucine and miglustat may be formulated and administered to a subject in accordance with known teachings in the art. For example, acetyl-leucine and miglustat may be formulated as separate pharmaceutical compositions. These pharmaceutical compositions may comprise the active agent, i.e., acetyl-leucine or miglustat, and one or more pharmaceutically acceptable carriers. Acetyl-leucine and miglustat may also be formulated as single pharmaceutical composition comprising both active agents and one or more pharmaceutically acceptable carriers.

The pharmaceutical compositions comprising acetyl-leucine and miglustat, either separately or together in a single composition, may take any of a number of different forms depending on the manner in which they are to be used. Thus, for example, the pharmaceutical compositions may be in the form of a powder, tablet, capsule, liquid, ointment, cream, gel, hydrogel, aerosol, spray, micellar solution, transdermal patch, liposome suspension, or any other suitable form that may be administered to a subject in need of treatment.

A "pharmaceutically acceptable carrier" as referred to herein, is any known compound or combination of known compounds, e.g., excipients, carriers, etc., that are known to those skilled in the art to be useful in formulating pharmaceutical compositions. It will be appreciated that the carrier of the pharmaceutical composition should be one which is tolerated by the subject to whom it is given.

In one embodiment, the pharmaceutically acceptable carrier may be a solid, and the composition may be in the form of a powder or tablet. A solid pharmaceutically acceptable carrier may include, but is not limited to, one or more substances which may also act as flavouring agents, buffers, lubricants, stabilisers, solubilisers, suspending agents, wetting agents, emulsifiers, dyes, fillers, glidants, compression aids, inert binders, sweeteners, preservatives, dyes, coatings, or tablet-disintegrating agents. The carrier may also be an encapsulating material. In powders, the carrier may be a finely divided solid that is in admixture with the finely divided active agents according to the invention. In tablets, the active agent may be mixed with a carrier having the necessary compression properties in suitable proportions and compacted in the shape and size desired. The powders and tablets may, for example, contain up to 99% of the active agents. Suitable solid carriers include, for example, calcium phosphate, magnesium stearate, talc, sugars, lactose, dextrin, starch, gelatin, cellulose, polyvinylpyrrolidine, low melting waxes and ion exchange resins. In another embodiment, the pharmaceutically acceptable carrier may be a gel and the composition may be in the form of a cream or the like.

The carrier may include, but is not limited to, one or more excipients or diluents. Examples of such excipients are gelatin, gum arabicum, lactose, microcrystalline cellulose, starch, sodium starch glycolate, calcium hydrogen phosphate, magnesium stearate, talcum, colloidal silicon dioxide, and the like.

In another embodiment, the pharmaceutically acceptable carrier may be a liquid. In one embodiment, the pharmaceutical composition is in the form of a solution. Liquid carriers are used in preparing solutions, suspensions, emulsions, syrups, elixirs and pressurized compositions. Acetyl-leucine and/or miglustat may be dissolved or suspended in a pharmaceutically acceptable liquid carrier such as water, an organic solvent, a mixture of both or pharmaceutically acceptable oils or fats. The liquid carrier may contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid carriers for oral and parenteral administration include water (partially containing additives as above, e.g. cellulose derivatives, such as sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier may also be an oily ester such as ethyl oleate and isopropyl myristate. Sterile liquid carriers are useful in sterile liquid form compositions for parenteral administration. The liquid carrier for pressurised compositions may be a halogenated hydrocarbon or other pharmaceutically acceptable propellant.

Liquid pharmaceutical compositions, which are sterile solutions or suspensions, may be utilised by, for example, intramuscular, intrathecal, epidural, intraperitoneal, intravenous and subcutaneous injection. The active agent may be prepared as a sterile solid composition that may be dissolved or suspended at the time of administration using sterile water, saline, or other appropriate sterile injectable medium.

The compositions may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The compositions may also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

In one embodiment, the pharmaceutical composition of both acetyl-leucine and miglustat is a solid oral dosage form, such as a tablet. In tablets, the active agent may be mixed with a vehicle, such as a pharmaceutically acceptable carrier, having the necessary compression properties in suitable proportions and compacted in the shape and size desired. The tablets may contain up to 99% by weight of the active agents.

Pharmaceutical compositions in solid oral dosage form, such as tablets, may be prepared by any method known in the art of pharmacy. Pharmaceutical compositions are usually prepared by mixing the active agent with conventional pharmaceutically acceptable carriers.

A tablet may be formulated as is known in the art. Tanganil^{®}, for example, includes wheat starch, pregelatinised maize (corn) starch, calcium carbonate and magnesium stearate as excipients. The same, or similar, excipients, for example, may be employed with the present disclosure.

The composition of each 700 mg Tanganil^{®} tablet is as follows: 500 mg acetyl-DL-leucine, 88 mg wheat starch, 88 mg pregelatinised maize (corn) starch, 13 mg calcium carbonate and 11 mg magnesium stearate. The same tablets, for example, may be employed in the methods of the present disclosure.

As discussed above, acetyl-leucine and miglustat may be formulated and administered as a pharmaceutical composition taking any number of different forms. For example, acetyl-leucine may be formulated as a pharmaceutical composition to facilitate its delivery across the blood-brain barrier. As a further example, acetyl-leucine may be formulated as a pharmaceutical composition for bypassing the blood-brain barrier.

In one embodiment, the pharmaceutical composition, e.g., comprising acetyl-L-leucine, or salt thereof, is formulated for nanodelivery, e.g., colloidal drug-carrier systems. Suitable examples include but are not limited to liposomes, nanoparticles (e.g., polymeric, lipid and inorganic nanoparticles), nanogels, dendrimers, micelles, nanoemulsions, polymersomes, exosomes, and quantum dots. See, e.g., Patel et al., "Crossing the Blood-Brain Barrier: Recent Advances in Drug Delivery to the Brain," CNS Drugs 31:109-133 (2017); Kabanov et al., "New Technologies for Drug Delivery across the Blood Brain Barrier," Curr Pharm Des., 10(12):1355-1363 (2004); Cheng et al., "Highly Stabilized Curcumin Nanoparticles Tested in an In Vitro Blood-Brain Barrier Model and in Alzheimer's Disease Tg2576 Mice," The AAPS Journal, vol. 15, no. 2, pp. 324-336 (2013); Lähde et al. "Production of L-Leucine Nanoparticles under Various Conditions Using an Aerosol Flow Reactor Method," Journal of Nanomaterials, vol. 2008, article ID 680897 (2008).

In one embodiment, the pharmaceutical composition, e.g., comprising N-acetyl-L-leucine, or salt thereof, is formulated for direct delivery to the central nervous system (CNS), such as by injection or infusion. Formulations for and methods of direct delivery to the CNS are known in the art. *See, e.g.,* U.S. Patent No. 9,283,181. Examples of such administration include but are not limited to intranasal, intraventricular, intrathecal, intracranial, and delivery via nasal mucosal grafting. In one embodiment, the pharmaceutical composition is administered by intracerebroventricular infusion.

In one embodiment, the pharmaceutical composition is formulated for (and administered by) intranasal delivery. See, e.g., Hanson et al., "Intranasal delivery bypasses the blood-brain barrier to target therapeutic agents to the central nervous system and treat neurodegenerative disease," BMC Neurosci. 9(Suppl 3):S5 (2008). In one embodiment, the pharmaceutical composition is formulated for (and administered by) delivery via a nasal mucosal graft. In one embodiment, the pharmaceutical composition is formulated for (and administered by) intracerebroventricular injection or infusion. In another embodiment, the pharmaceutical composition is formulated for (and administered by) intrathecal intracisternal injection or infusion. In one embodiment, the pharmaceutical composition is formulated for (and administered by) intrathecal lumbar injection or infusion.

Various techniques may be used including, without limitation, injection through a burrhole or cisternal or lumbar puncture or the like as known in the art. Various devices, whether internal (e.g., implanted) or external, may be used for delivery as known in the art, such as pumps, catheters, reservoirs, etc. In one embodiment, the administration interval is once every two weeks.

"Lysosomal storage disease" or "LSD" refers to any disorder that involves dysfunction or disruption in the late endosomal/lysosomal system and the accumulation of undigested or partially digested macromolecules. The LSD may involve increased storage of lipids or non-lipids. All LSDs are within the scope of the present disclosure.

In one embodiment, the LSD is chosen from any of glycogen storage disease, mucopolysaccaridoses, mucolipidoses, oligosaccharidoses, lipidoses, sphingolipidoses, and lysosomal transport diseases.

The glycogen storage disease may be chosen from Infantile-onset Pompe disease, Lateonset Pompe disease and Danon disease.

The mucopolysaccharidoses may be chosen from any of MPS IH, MPS I H-S, MPS IS, MPS IIA, MPS IIB, MPS IIIA-D, MPS IVA, MPS VI, MPS VII and MPS IX. In one embodiment, the mucopolysaccharidosis is MPS III or MPS VII. In one embodiment, the mucopolysaccharidosis is MPS IIIB.

The mucolipidoses may be chosen from any of mucolipidosis I, mucolipidosis II, mucolipidosis III, and mucolipidosis IV. In one embodiment the mucolipidosis is mucolipidosis II or mucolipidosis III.

The oligosaccharidoses may be chosen from any of beta-mannosidosis, alphafucosidosis, and aspartylglucosaminuria. In one embodiment, the oligosaccharidosis is aspartylglucosaminuria.

The lipidoses may be chosen from any of Niemann-Pick disease type C, Niemann-Pick disease type D, neuronal ceroid lipofuscinoses (Type I to X inclusive), and Wolman disease. In one embodiment, the lipidosis is Niemann-Pick disease type C.

The sphingolipidoses may be chosen from any of Niemann-Pick disease type A/B, Gaucher disease types I, II, and III, Krabbe disease, Fabry disease, Schindler Disease, GM1 gangliosidosis, Morquio B disease, GM2 gangliosidoses, metachromatic leukodystrophy, Farber disease, multiple sulfatase deficiency, lysosomal acid lipase deficiency and galactosialidosis. In one embodiment, the sphingolipidoses are chosen from Niemann-Pick disease type A, GM1 gangliosidosis, Tay-Sachs disease, the AB variant of Tay-Sachs disease, and Sandhoff disease.

The lysosomal transport diseases may be chosen from cystinosis, pycnodysostosis, sialic acid storage disease and infantile free sialic acid storage disease.

The LSD may be a primary lysosomal hydrolase defect, a post-translational processing defect of lysosomal enzymes, a trafficking defect for lysosomal enzymes, a defect in lysosomal enzyme protection, a defect in soluble non-enzymatic lysosomal proteins, a transmembrane (non-enzyme) protein defect or an unclassified defect.

In one embodiment, the LSD is chosen from a primary lysosomal hydrolase defect. Primary lysosomal hydrolase defects include, but are not limited to, Tay-Sachs disease (β-hexosaminidase A defect), Sandhoff disease (β-hexosaminidase A+B defect), Fabry disease (α-galactosidase A defect), Krabbe disease (β-galactosyl ceramidase defect), Niemann-Pick Type A and B (sphingomyelinase defect), metachromatic leukodystrophy (arylsulphatase A defect), MPS IH (Hurler syndrome; α-iduronidase defect), MPS IS (Scheie syndrome; α-iduronidase defect), MPS IH-S (Hurler-Scheie syndrome; α-iduronidase defect), MPS II (Hunter syndrome; iduronate sulphatase defect), MPS IIIA (Sanfilippo A syndrome; heparan sulphamidase defect), MPS IIIB (Sanfilippo B syndrome; acetyl α-glucosaminidase defect), MPS IIIC (Sanfilippo C syndrome; acetyl CoA: α-glucosaminide N-acetyltransferase defect), MPS IIID (Sanfilippo D syndrome; N-acetyl glucosamine-6-sulphatase defect), MPS IV A (Morquio A disease; acetyl galactosamine-6-sulphatase defect), MPS IVB (Morquio B disease; β-galactosidase defect), MPS V (redesignated MPS IS), MPS VI (Maroteaux Lamy Syndrome; acetyl galactosamine-4-sulphatase (arylsulphatase B) defect), MPS VII (Sly Syndrome; β-glucuronidase defect), MPS IX (hyaluronidase defect), Wolman/cholesteryl ester storage disease (WD; acid lipase defect), Pompe disease (Type II; α-1,4-glucosidase defect), aspartylglucosaminuria (glycosylasparaginase defect), fucosidosis (α-fucosidase defect), α-mannosidosis (α-mannosidase defect), β-mannosidosis 5 (β-mannosidase defect), Schindler disease (N-acetylgalactosaminidase defect), sialidosis/ML I (α-neuraminidase defect), infantile neuronal ceroid lipofuscinosis (CLN1; palmitoyl protein thioesterase defect), late infantile neuronal ceroid lipofuscinosis (CLN2; carboxypeptidase defect), early infantile GM1 gangliosidosis, late infantile GM1 gangliosidosis, adult infantile GM1 gangliosidosis, Gaucher Disease Type 1 (Non-Neuronopathic), Gaucher Disease Type 2/3 (Neuronopathic), Neuronal Ceroid Lipofuscinosis Type 4 (CLN4; Kufs disease; Adult NCL; palmotoyl-protein thioesterase-1 deficiency (Type A); Cathepsin F deficiency (Type B)), Neuronal Ceroid Lipofuscinosis Type 10 (CLN10; Congenital Cathepsin D Deficiency), Pycnodysostosis (Cathepsin K defect), Infantile-Onset Pompe Disease, Late-Onset Pompe Disease, Farber Disease (Farber's lipogranulomatosis; ceramidase deficiency; Fibrocytic dysmucopolysaccharidosis; Lipogranulomatosis) and Galactosialidosis (protective protein cathepsin A defect, PPCA defect). In one embodiment, the primary lysosomal hydrolase defect is chosen from Tay-Sachs disease, Sandhoff disease, Niemann-Pick Type A, Niemann-Pick Type B, neuronal ceroid lipofuscinoses, Gaucher disease, Fabry disease, Krabbe disease, GM1 gangliosidosis, GM2 gangliosidosis, metachromatic leukodystrophy, and Farber disease. In one embodiment, the primary lysosomal hydrolase defect is chosen from Tay-Sachs disease, Sandhoff disease, Niemann-Pick Type A, Niemann-Pick Type B, and GM1 gangliosidosis.

In one embodiment, the LSD is NPC.

In one embodiment, the LSD is Gaucher disease.

In one embodiment, the LSD is GM1 gangliosidosis.

In one embodiment, the LSD is GM1 gangliosidosis.

The term "acetyl-leucine" refers collectively to N-acetyl-DL-leucine (ADLL), or a pharmaceutically acceptable salt thereof; N-acetyl-D-leucine (ADL), or a pharmaceutically acceptable salt thereof; and N-acetyl-L-leucine (ALL), or a pharmaceutically acceptable salt thereof. The term acetyl-leucine includes isotopically-labelled analogs of N-acetyl-DL-leucine, N-acetyl-D-leucine, and N-acetyl-L-leucine, wherein one or more atoms are replaced by an atom having a different atomic mass or mass number. Examples of isotopes that can be incorporated include isotopes of hydrogen, carbon, nitrogen, and oxygen, such as ²H (or deuterium (D)), ³H, ¹¹C, ¹³C, ¹⁴C, ¹⁵N, ¹⁸O, and ¹⁷O. In one embodiment, provided is an isotopically-labelled analog of acetyl-leucine, wherein substantially all of the atoms at a position within acetyl-leucine are replaced by an atom having a different atomic mass or mass number. In another embodiment, provided is an isotopically-labelled analog of acetyl-leucine, wherein a portion of the atoms at a position within acetyl-leucine are replaced, e.g., acetyl-leucine is enriched at one or more positions with an atom having a different atomic mass or mass number. Isotopically-labelled acetyl-leucine can be prepared by methods known in the art.

In one embodiment, the N-acetyl-DL-leucine, N-acetyl-D-leucine, or N-acetyl-L-leucine is not isotopically-labelled.

In one embodiment, the isotopically-labelled analog is a deuterated analog of N-acetyl-DL-leucine, N-acetyl-D-leucine, or N-acetyl-L-leucine, wherein one or more hydrogen atoms are replaced with deuterium. In one embodiment, one hydrogen atom of N-acetyl-DL-leucine, N-acetyl-D-leucine, or N-acetyl-L-leucine is replaced with deuterium. In another embodiment, two hydrogen atoms of N-acetyl-DL-leucine, N-acetyl-D-leucine, or N-acetyl-L-leucine are replaced with deuterium. In another embodiment, three hydrogen atoms of N-acetyl-DL-leucine, N-acetyl-D-leucine, or N-acetyl-L-leucine are replaced with deuterium. In another embodiment, four hydrogen atoms of N-acetyl-DL-leucine, N-acetyl-D-leucine, or N-acetyl-L-leucine are replaced with deuterium. In another embodiment, five hydrogen atoms of N-acetyl-DL-leucine, N-acetyl-D-leucine, or N-acetyl-L-leucine are replaced with deuterium. In another embodiment, six hydrogen atoms of N-acetyl-DL-leucine, N-acetyl-D-leucine, or N-acetyl-L-leucine are replaced with deuterium.

In one embodiment, the acetyl-leucine used in the methods of the present disclosure is N-acetyl-DL-leucine, or a deuterated analog thereof. In another embodiment, the acetyl-leucine used in the methods of the present disclosure is N-acetyl-D-leucine, or a deuterated analog thereof. In another embodiment, the acetyl-leucine used in the methods of the present disclosure is N-acetyl-L-leucine, or a deuterated analog thereof.

In another embodiment, the acetyl-leucine used in the methods of the present disclosure is N-acetyl-DL-leucine. In another embodiment, the acetyl-leucine used in the methods of the present disclosure is N-acetyl-D-leucine. In another embodiment, the acetyl-leucine used in the methods of the present disclosure is N-acetyl-L-leucine.

"Miglustat" refers to 1,5-(butylimino)-1,5-dideoxy-D-glucitol (also known as N-butyl-deoxynojirimycin).

"Administered in combination" and similar phrases mean that two agents, e.g., (1) acetyl-DL-leucine, acetyl-D-leucine, or acetyl-L-leucine; and (2) miglustat, are administered concurrently to the subject being treated. In one embodiment, acetyl-DL-leucine, acetyl-D-leucine, or acetyl-L-leucine and miglustat are administered in combination as a first line therapy to treat a LSD.

"First line therapy" means a treatment regimen generally accepted or recommended by the medical establishment or a regulatory authority, e.g., the U.S. Food and Drug Administration or the European Medicines Agency, for the initial treatment of a condition, disease, or disorder.

"Concurrently" means that each active agent is administered either (i) simultaneously; or (ii) sequentially in any order at different points in time. A combination of two agents is considered to be administered simultaneously if each agent is administered to the subject less than 1 minute apart. If not administered simultaneously, it is meant that both agents are administered to a subject in a sequence and sufficiently close in time so as to provide the desired therapeutic effect and can act in concert to treat a LSD.

In one embodiment, acetyl-leucine and miglustat are administered separately, in any appropriate form and by any suitable route. In one embodiment, both acetyl-leucine and miglustat are orally administered to the subject as tablets.

In one embodiment, acetyl-leucine is administered to the subject 1 minute to 24 hours before the administration of miglustat to the subject. For example, acetyl-leucine is administered 5 minutes, 10 minutes, 15 minutes, 30 minutes, 45 minutes, 1 hour, 2 hours, 4 hours, 6 hours, or 12 hours before the administration of miglustat to a subject.

In another embodiment, acetyl-leucine is administered simultaneously with miglustat to the subject.

In another embodiment, acetyl-leucine is administered to the subject 1 minute to 24 hours after the administration of miglustat to the subject. For example, acetyl-leucine is administered 5 minutes, 10 minutes, 15 minutes, 30 minutes, 45 minutes, 1 hour, 2 hours, 4 hours, 6 hours, or 12 hours after, the administration of miglustat to a subject.

In another embodiment, acetyl-leucine and miglustat are administered to the subject about 1 minute to about 24 hours apart. For example, acetyl-leucine and miglustat are administered about 1 minute apart, 5 minutes apart, 10 minutes apart, 30 minutes apart, 45 minutes apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, or 11 hours to 12 hours apart.

"Naive to treatment with miglustat" and similar phrases means that miglustat has not previously been administered to the subject to treat a LSD.

"Subject" means a human.

"Subject in need thereof" means a human who has a LSD and is need of treatment. The subject may or may not have been diagnosed a LSD. For example, the subject may not yet have a diagnosis (clinical or otherwise) of a LSD, but may have one or more symptoms of a LSD. The subject may also have a biochemical or other similar identifiable marker of a LSD.

A "therapeutically effective amount" of acetyl-leucine or miglustat is any amount of either active agent which, when administered to a subject, is the amount that is needed to produce the desired effect, which, for the present disclosure, can be therapeutic and/or prophylactic. The dose may be determined according to various parameters, such as the specific active agent used; the age, weight and condition of the patient to be treated; the route of administration; and the required regimen. A physician will be able to determine the required route of administration and dosage for any particular patient. For example, a daily dose of either active agent may be from about 10 to about 225 mg per kg, from about 10 to about 150 mg per kg, or from about 10 to about 100 mg per kg of body weight.

As used herein, "treating" or "treatment" refers to any indicia of success in preventing, arresting, or ameliorating a disease, disorder, condition, or syndrome, e.g., a LSD, in a subject, and/or preventing, arresting, or ameliorating any one or more symptoms a disease, disorder, condition, or syndrome, e.g., a LSD, in a subject, including any objective or subjective parameter such as abatement; remission; preventing, diminishing, inhibiting, or eliminating one or more symptoms; making the disease, disorder, condition, or syndrome more tolerable to the subject; slowing in the worsening of the disease, disorder, condition, or syndrome; or improving the physical or mental well-being of the subject in need thereof.

The terms "treating" or "treatment" also encompasses inducing inhibition, regression, or stasis of the disease, disorder, condition, or syndrome, e.g., a LSD. For example, treatment of a patient or subject in need of treatment for a LSD includes reducing a symptom of the LSD in the subject, inducing clinical response, inhibiting or reducing progression of the LSD, or inhibiting or reducing a complication of the LSD.

Preventing, arresting, or ameliorating an injury or pathology of a disease, disorder, condition, or syndrome, e.g., a LSD, e.g., NPC, such as preventing, diminishing, inhibiting, or eliminating one or more symptoms of disease, disorder, condition, or syndrome can be based on objective and/or subjective parameters, including, e.g., the results of physical examination(s), neurological examination(s), and/or psychiatric evaluation(s). The success of treatment for certain diseases e.g., a LSD, may be measured or evaluated by, for example, comparing the severity of the disease, e.g., objective and/or subjective parameters of the LSD, e.g., NPC, before treatment with acetyl-leucine and miglustat is initiated, with the severity of the disease following the initiation of treatment with acetyl-leucine and miglustat. For example, the severity of NPC may be assessed using a scale, index, rating, or score. In one embodiment, the treatment described herein improves such an assessment from a value or degree characteristic of a symptomatic subject to a value or degree characteristic of a non-symptomatic subject. In one embodiment, the treatment described herein improves such an assessment compared to a baseline. The baseline may be, for example, the subject's condition before initiating any treatment for the disease, e.g., NPC, or before initiating treatment for the disease with a acetyl-leucine and miglustat. Alternatively, the baseline may be, for example, the subject's condition after a certain time period on treatment for the disease. In one embodiment, treatment with acetyl-leucine and miglustat as described herein improves the subject's assessment (e.g., scale, index, rating, or score of objective and/or subjective parameters) compared to a baseline by at least 10%, at least 20%, at least 30%, at least 40%, or at least 50%. In one embodiment, assessment is improved by at least 60%, at least 70%, at least 80%, at least 90%, or 100%.

In one embodiment, acetyl-leucine is administered at a dose ranging from about 500 mg to about 30 g per day. For example acetyl-leucine is administered at a dose ranging from about 500 mg to about 15 g per day, a dose ranging from about 1.5 g to about 10 g per day, optionally by solid oral or liquid oral route. N-Acetyl-DL-leucine, may be administered, for example, in a dose according to that of Tanganil^{®}, which is prescribed to adults in a dose of 1.5 g to 2 g per day, 3-4 tablets in two doses, morning and evening.

If a single enantiomer of acetyl-leucine, i.e., N-acetyl-D-leucine or N-acetyl-L-leucine, is administered the doses may be reduced accordingly. For instance, if only N-acetyl-L-leucine or if only N-acetyl-D-leucine is administered, the dose may range from about 250 mg to about 15 g per day, range from about 250 mg to about 10 g per day, or range from about 250 mg to about 5 g per day, such as from about 0.75 g to about 5 g per day.

In one embodiment, the administered dose ranges of acetyl-leucine are from about 1 g to about 30 g per day. For example, the administered dose ranges of acetyl-leucine are from about 1 g to about 15 g per day, from about 1 g to about 10 g per day, or from about 1.5 g to about 7 g per day, from 15.1 g to about 30 g per day, 16 g to about 30 g per day, 17 g to about 30 g per day, 18 g to about 30 g per day, 19 g to about 30 g per day, or 20 g to about 30 g per day. It may be from about 2, 3, 4, 5, 6, 7, 8, 9, 10, 11, 12, 13, or 14 g to about 15 g per day. It may be from about 2, 3, 4, 5, 6, 7, 8 or 9 g to about 10 g per day. It may be from 15.1, 16, 17, 18, 19, 20, 21, 22, 23, 24, 25, 25, 27, 28, or 29 g to about 30 g per day. It may be more than about 1.5 g per day, but less than about 15, 14, 13, 12, 11, 10, 9, 8, 7, 6 or 5 g per day. In one embodiment, the dose ranges from about 4 g to about 6 g per day. In one embodiment, the dose ranges from about 4 g to about 5 g per day. In one embodiment, the dose is about 4.5 g per day. In one embodiment, the dose is about 5 g per day. In one embodiment, the dose is about 1 g per day, about 2 g per day, about 3 g per day, about 4 g per day, about 5 g per day, about 6 g per day, about 7 g per day, about 8 g per day, about 9 g per day, about 10 g per day, about 11 g per day, about 12 g per day, about 13 g per day, about 14 g per day, or about 15 g per day. In another embodiment, the dose is about 16 g per day, about 17 g per day, about 18 g per day, about 19 g per day, or about 20 g per day. In another embodiment, the dose is about 21 g per day, about 22 g per day, about 23 g per day, about 24 g per day, about 25 g per day, about 26 g per day, about 27 g per day, about 28 g per day, about 29 g per day, or about 30 g per day. In one embodiment, these doses are administered in a solid oral dosage form, notably tablets. In another embodiment, these doses are for acetyl-leucine when in its racemic form. Doses for acetyl-leucine when an enantiomeric excess is present may be lower, for example, around 50% lower. The above recited dose-ranges when halved are thus also explicitly encompassed by the present disclosure.

In one embodiment, miglustat is administered at a dose ranging from about 0.01 g to about 5 g per day. In another embodiment, miglustat is administered at a dose of about 0.1 g per day. In another embodiment, miglustat is administered at a dose of about 0.2 g per day. In another embodiment, miglustat is administered at a dose of about 0.3 g per day. In another embodiment, miglustat is administered at a dose of about 0.4 g per day. In another embodiment, miglustat is administered at a dose of about 0.5 g per day. In another embodiment, miglustat is administered at a dose of about 0.6 g per day. In another embodiment, miglustat is administered at a dose of about 0.7 g per day. In another embodiment, miglustat is administered at a dose of about 0.8 g per day. In another embodiment, miglustat is administered at a dose of about 0.9 g per day. In another embodiment, miglustat is administered at a dose of about 1 g per day.

In one embodiment, the total daily dose of acetyl-leucine may be spread across multiple administrations, i.e., administration may occur two or more times a day to achieve the total daily dose. As an example, the required number of tablets to provide the total daily dose of a acetyl-leucine may be split across two administrations (for example, in the morning and evening) or three administrations (for example, in the morning, noon and evening). Each dose may be suitably administered with or without food. For example, N-acetyl-L-leucine or N-acetyl-DL-leucine may be dosed by about 1 or about 2 hours before meals, such as at least about 20 minutes, at least about 30 minutes, at least about 40 minutes, or at least about 1 hour before meals, or may be dosed by about 1, about 2, or about 3 hours after meals, such as waiting at least about 20 minutes, at least about 30 minutes, at least about 1 hour, at least about 1.5 hours, at least about 2 hours, or at least about 2.5 hours after meals. For example, a total daily dose of 4.5 g acetyl-DL-leucine may be administered as three Tanganil^{®} (or equivalent) tablets before, with, or after breakfast, three further tablets before, with, or after lunch and three further tablets before, with, or after dinner.

The treatment duration for the combination of acetyl-leucine and miglustat may be about seven days or more. For example, the treatment duration may be about two weeks or more, about three weeks or more, about one month or more, about six weeks or more, about seven weeks or more, or about two months or more. In one embodiment, the treatment duration is about three months or more, e.g., about four months or more, about five months or more or about six months or more. The treatment duration may also be about 1 year or more, about 2 years or more, about 4 years or more, about 5 years or more, or about 10 years or more. The treatment duration may also be the life-time of the subject.

Any and all combinations of dosage form, dose amount, dosing schedule, and treatment duration for the combination of acetyl-leucine and miglustat are envisaged and encompassed by the disclosure. In one embodiment, the dose of acetyl-leucine is from about 4 g to about 10 g per day, taken across one, two, or three administrations per day, for a treatment duration of about two months or more. In another embodiment, the dose of acetyl-leucine is more than 4 g but no more than 5 g per day, taken across one, two, or three administrations per day, for a treatment duration of about six months or more. The dosage form may be a solid oral dosage form, notably tablets.

In one embodiment, the combination of acetyl-leucine and miglustat is used for treating a LSD or one or more symptoms of a LSD. As used herein, "treating a LSD or one or more symptoms of a LSD" and the like refer to delaying onset of a LSD or one or more symptoms of a LSD that would otherwise be expected to manifest according to typical disease progression, reducing the severity of a LSD or reducing the severity of or eliminating one or more existing symptoms associated with a LSD, delaying progression of a LSD or one or more symptoms of a LSD over time as compared to typical disease progression, and/or reversing progression of a LSD or one or more symptoms of a LSD over time. "Treating a LSD or one or more symptoms of a LSD" may also refer to improving a biochemical marker of a LSD.

As used herein, "typical disease progression," "disease progression that would typically be expected" and the like refer to the typical or expected progression of a LSD, one or more symptoms associated with a LSD, or a biochemical marker of a LSD if the subject were untreated. Typical or expected disease progression may be based, for example, on a known scale, index, rating, or score, or other suitable test, for assessing the progression of a LSD, one or more symptoms associated with a LSD, or a biochemical marker of a LSD, such as those described herein. The scale, index, rating, score, or other suitable test may correspond to the progression of the LSD overall or to the progression of one or more symptoms associated with the LSD. For instance, typical or expected disease progression may be based on the typical or expected onset or severity of the LSD or a symptom or collection of symptoms associated with the LSD. The typical or expected disease progression may be determined on a subject-by-subject basis or may be based on what is typically observed for or experienced by a collection of subjects afflicted with the LSD, such as a population or subpopulation of subjects. Subpopulations may include, for example, subpopulations of the same gender, of the same or similar age, of the same or similar timing for the onset of one or more symptoms, etc.

In another embodiment, "treating a LSD or one or more symptoms of a LSD" refers to delaying onset of a LSD or one or more symptoms of a LSD that would otherwise be expected to manifest according to typical disease progression. As used herein, "delaying onset of a LSD or one or more symptoms of a LSD" and the like refer to increasing the time to, or preventing, onset of the LSD or one or more symptoms of the LSD. For example, onset can be said to be delayed when the time to manifestation of a LSD or one or more symptoms of a LSD takes at least 5% longer than that observed according to typical disease progression. Further for example, an increase in time of at least 10%, at least 15%, at least 20%, at least 25%, at least 30%, at least 40%, at least 50%, at least 60%, at least 70%, at least 80%, at least 90% or at least 100% is observed. In one embodiment, the subject is asymptomatic. The administration of acetyl-leucine and miglustat may be initiated at the time the subject is asymptomatic to delay onset of a LSD or one or more symptoms of a LSD that would otherwise be expected to manifest according to typical disease progression. In another embodiment, the subject is symptomatic. The administration of acetyl-leucine and miglustat may be initiated at the time the subject has some symptoms in order to delay onset of one or more additional symptoms of a LSD that would otherwise be expected to manifest according to typical disease progression. The subject in need thereof may continue to receive treatment with acetyl-leucine and miglustat in accordance with the durations described herein. In one embodiment, the treatment prevents onset of one or more symptoms of the LSD that would otherwise be expected to manifest according to typical disease progression.

In another embodiment, "treating a LSD or one or more symptoms of a LSD" refers to reducing the severity of a LSD or reducing the severity of or eliminating one or more existing symptoms associated with a LSD. The severity of a LSD or of the existing symptom(s) may be assessed using a known scale, index, rating, or score, such as those described as examples herein, or another suitable test for assessing severity. For example, the scale, index, rating, score, or other suitable test may correspond to the severity of the LSD overall or to the severity of one or more symptoms associated with the LSD. In one embodiment, the treatment improves such an assessment from a value or degree characteristic of a symptomatic patient to a value or degree characteristic of a non-symptomatic patient.

In another embodiment, "treating a LSD or one or more symptoms of a LSD" refers to delaying progression of a LSD or one or more symptoms associated with a LSD over time as compared to typical disease progression, or reversing progression of a LSD or one or more symptoms associated with a LSD over time. The time over which the treatment delays or reverses progression may coincide with the duration of treatment as described herein. The treatment may delay or reverse progression over a duration of, for example, about seven days or more, about two weeks or more, about three weeks or more, about one month or more, about six weeks or more, about seven weeks or more or about two months or more. For example, the treatment delays or reverses progression over a duration of about three months or more, about four months or more, about five months or more or about six months or more. Further for example, it delays or reverses progression over a duration of about 1 year or more, about 2 years or more, about 3 years or more, about 4 years or more, about 5 years or more, or about 10 years or more. The treatment may delay or reverse progression of the LSD or one or more symptoms associated with the LSD over the lifetime of the patient.

In another embodiment, "treating a LSD or one or more symptoms of a LSD" refers to delaying progression of a LSD or one or more symptoms of a LSD over time as compared to typical disease progression. As used herein, "delaying progression of a LSD or one or more symptoms associated with a LSD over time" and the like refer to slowing and/or stopping progression of the LSD or one or more symptoms of the LSD (*e.g.,* slowing and/or stopping the worsening or increasing severity of the LSD or one or more symptoms of the LSD) over time. Disease progression may be determined, for example, using a known scale, index, rating, or score, such as those described as examples herein, or other suitable tests for assessing progression. For example, the scale, index, rating, score, or other suitable test may correspond to the progression of the LSD overall or to the progression of one or more symptoms associated with the LSD. In one embodiment, "delaying progression of a LSD or one or more symptoms associated with a LSD" means that a subject's disease severity value (e.g., overall severity or severity of one or more symptoms) determined by a known scale, index, rating, score, etc., or another suitable test for evaluating severity, does not meaningfully increase (e.g., at least remains substantially constant). In one embodiment, "delaying progression of a LSD or one or more symptoms of a LSD" means preventing the subject from reaching, or increasing the time taken for a subject to reach (e.g., decreasing the rate of change of increasing severity), a severity value according to a known scale, index, rating, score, etc., or other suitable test, for assessing progression compared to a value corresponding to typical disease progression. For example, progression can be said to be delayed when the time to reach a severity value takes at least 5% longer than that observed according to typical disease progression. Further for example, an increase in time of at least 10%, at least 15%, at least 20%, at least 25%, at least 30%, at least 40%, at least 50%, at least 60%, at least 70%, at least 80%, at least 90% or at least 100% is observed. The time over which the treatment delays progression of a LSD or one or more symptoms of a LSD may coincide with the duration of treatment as described herein. In one embodiment, the treatment delays progression for at least about three months, at least about four months, at least about five months, or at least about six months. In another embodiment, the treatment delays progression for at least about 1 year, at least about 2 years, at least about 3 years, at least about 4 years, at least about 5 years, or at least about 10 years. The treatment may delay progression over the lifetime of the patient.

In another embodiment, "treating a LSD or one or more symptoms of a LSD" refers to reversing progression of a LSD or one or more symptoms of a LSD over time. As used herein, "reversing progression of a LSD or one or more symptoms of a LSD over time" and the like refer to stopping progression and reducing the severity of the LSD or one or more symptoms of the LSD over time. Disease progression and severity may be determined, for example, using a known scale, index, rating, or score, such as those described as examples herein, or another suitable test for assessing progression and severity. For example, the scale, index, rating, score, or other suitable test may correspond to the progression and severity of the LSD overall or to the progression and severity of one or more symptoms associated with the LSD. In one embodiment, "reversing progression of a LSD or one or more symptoms of a LSD over time" means that a subject's disease severity value (e.g., overall severity or severity of one or more symptoms) determined by a known scale, index, rating, score, etc., or another suitable test, for evaluating severity, improves over time (i.e., shows a reduction in severity over time). The time over which the treatment reverses progression of a LSD or one or more symptoms of a LSD may coincide with the duration of treatment as described herein. In one embodiment, the treatment reverses progression for at least about three months, at least about four months, at least about five months, or at least about six months. In another embodiment, the treatment reverses progression for at least about 1 year, at least about 2 years, at least about 3 years, at least about 4 years, at least about 5 years, or at least about 10 years. The treatment may reverse progression over the lifetime of the patient.

In another embodiment, "treating a LSD or one or more symptoms of a LSD" refers to improving in the subject a biochemical marker of a LSD (e.g., increased levels of the storage metabolite(s) or secondary biochemical changes resulting from the primary storage). A biochemical marker is a signal of disease activity and may provide ongoing indications of disease severity and progression over time. In one embodiment, the biochemical marker is improved in view of a control value. In one embodiment, the biochemical marker is chosen from increased lysosomal volume and increased glycosphingolipid (GSL) levels. In one embodiment, the biochemical marker is increased lysosomal volume and the treatment reduces lysosomal volume in the subject. In one embodiment, the biochemical marker is increased glycosphingolipid (GSL) levels and the treatment reduces GSL levels in the subject. In one embodiment, the treatment improves a biochemical marker over time. For example, in one embodiment, improving a biochemical marker over time means that the treatment improves a biochemical marker over time toward a control value, prevents the progression of a biochemical marker over time, and/or delays the progression of the biochemical marker over time as compared to typical disease progression. The time over which the treatment improves a biochemical marker may coincide with the duration of treatment as described herein. In one embodiment, the treatment improves a biochemical marker for at least about three months, at least about four months, at least about five months, or at least about six months. In a further embodiment, the treatment improves a biochemical marker for at least about 1 year, at least about 2 years, at least about 3 years, at least about 4 years, at least about 5 years, or at least about 10 years. The treatment may improve the biochemical marker over the lifetime of the patient.

A "symptom" of a LSD includes any clinical or laboratory manifestation associated with a LSD and is not limited to what the subject can feel or observe. Symptoms as described herein include, but are not limited to, neurological symptoms and psychiatric symptoms. Examples of neurological symptoms include ataxia, other movement disorders such as hypokinesia, rigor, tremor or dystonia, central ocular motor disorders such as vertical and horizontal supranuclear saccade/gaze palsy and neuropsychological deficits such as dementia. Examples of psychiatric symptoms include depression, behavioural disorders or psychosis. Onset of symptoms may range from birth to adulthood.

Progression of a LSD or one or more symptoms of a LSD over time or through treatment can be monitored, for example, using one or more known tests at two or more time points and comparing the results. Disease progression and/or severity can be assessed, for example, using the Scale for the Assessment and Rating of Ataxia (SARA), Spinocerebellar Ataxia Functional Index (SCAFI), the International Cooperative Ataxia Rating Scale (ICARS), the brief ataxia rating scale (BARS), the modified Disability Rating Scale (mDRS), EuroQol 5Q-5D-5L (EQ-5D-5L), the visual analogue scale (VAS), Wechsler Adult Intelligence Scale-Revised (WAIS-R), Wechsler Intelligence Scale for Children-IV (WISC-IV), Montreal Cognitive Assessment (MoCA) or other suitable tests. For certain LSDs, such as NPC, particular scores have been developed and validated over the last decades, for instance the clinical severity score (CSS) and annual severity increment score (ASIS) (*see* Yanjanin et al., "Linear Clinical Progression, Independent of Age of Onset, in Niemann-Pick Disease, Type C," *Am J Med Genet Part B* 153B:132-140) and the modified 6-Domain NP-C disability Scale (mDRS score). For example, an NPC patient's severity can be quantified by assigning a CSS, which assesses various parameters of the disease (ambulation, seizures, eye movement, etc.) and gives each parameter a score out of 5. A higher score equals a greater severity. The ASIS quantifies the annual rate of change in the CSS, calculated by dividing the CSS by the patient's age. In this regard, certain scores in these tests are characteristic of symptomatic LSD patients and evidence disease progression and/or severity.

Thus, "treating a LSD or one or more symptoms of a LSD," for example, may be equated to achieving an improved assessment, such as those described herein, of a SARA, SCAFI, ICARS, BARS, mDRS, EQ-5D-5L, VAS, WAIS-R, WISC-IV, CSS and/or MoCA score, or result of another test suitable for characterising a LSD subject. For example, in one embodiment, "reducing the severity of a LSD or reducing the severity of or eliminating one or more existing symptoms of a LSD" means improving a SARA, SCAFI, ICARS, BARS, mDRS, EQ-5D-5L, VAS, WAIS-R, WISC-IV, CSS and/or MoCA score, or a result of another suitable test for evaluating severity, such as improving the score or result from a severity value characteristic of a symptomatic subject to a value characteristic of a non-symptomatic subject. In another embodiment, "delaying progression of a LSD or one or more symptoms of a LSD" means that a subject's SARA, SCAFI, ICARS, BARS, mDRS, EQ-5D-5L, VAS, WAIS-R, WISC-IV, CSS and/or MoCA score, or a result of another suitable test for evaluating progression, does not meaningfully increase (e.g., at least remains substantially constant). In a further embodiment, "delaying progression of a LSD or one or more symptoms of a LSD" means preventing a subject's SARA, SCAFI, ICARS, BARS, mDRS, EQ-5D-5L, VAS, WAIS-R, WISC-IV, CSS and/or MoCA score, or a result of another suitable test for evaluating progression, from reaching, or increasing the time taken to reach, a value compared to that of typical disease progression. In another embodiment, "reversing progression of a LSD or one or more symptoms of a LSD over time" means that a subject's SARA, SCAFI, ICARS, BARS, mDRS, EQ-5D-5L, VAS, WAIS-R, WISC-IV, CSS and/or MoCA score, or result of another suitable test for evaluating progression, improves over time (i.e., shows a reduction in severity over time).

For example, to evaluate overall neurological status, mDRS, a four-domain scale (ambulation, manipulation, language and swallowing), may be applied. Cerebellar function may be evaluated using SARA, an eight-item clinical rating scale (gait, stance, sitting, speech, fine motor function and taxis; range 0-40, where 0 is the best neurological status and 40 the worst), and SCAFI, comprising the 8-m-Walking-Time (8MW; performed by having patients walking twice as quickly as possible from one line to another excluding turning), 9-Hole-Peg-Test (9HPT) and the number of "PATA" repetitions over 10 s. Subjective impairment and quality of life may be evaluated using the EQ-5D-5L questionnaire and VAS. To assess ocular motor function, 3-dimensional videooculography (EyeSeeCam) may be used to measure the peak velocity of saccades, gain of smooth pursuit, peak slow phase velocity of gaze-evoked nystagmus (gaze-holding function), peak slow phase velocity of optokinetic nystagmus, and gain of horizontal vestibulo-ocular reflex. To evaluate the cognitive state, WAIS-R or WISC-IV, and MoCA, assessing different cognitive domains, including attention and concentration, executive functions, memory, language, visuoconstructional skills, conceptual thinking, calculations, and orientation with a maximum of 30 points and a cut-off score of 26, may be used. The skilled person will know how to perform these and other such tests.

### EXAMPLES

### GENERAL METHODS

### Animals and treatments

BALBc/NPC^{nih} and Sandhoff mice were bred as heterozygotes to generate affected mice (*Npc1*^{*-*/}*⁻ Hexb*^{*-*/}*⁻)* and controls (*Npc1*^{*+*/}*⁺ Hexb*^{*+*/*+*}). The mice were housed under non-sterile conditions, with food and water available *ad libitum.* All experiments were conducted using protocols approved by the UK Home Office Animal Scientific Procedures Act, 1986.

### Cells

Wild type and NPC1 null Chinese Hamster Ovary (CHO) cells were kindly provided by Chris Wassif (NIH).

### Antibodies

The antibodies used in this study are summarised in Table 2.

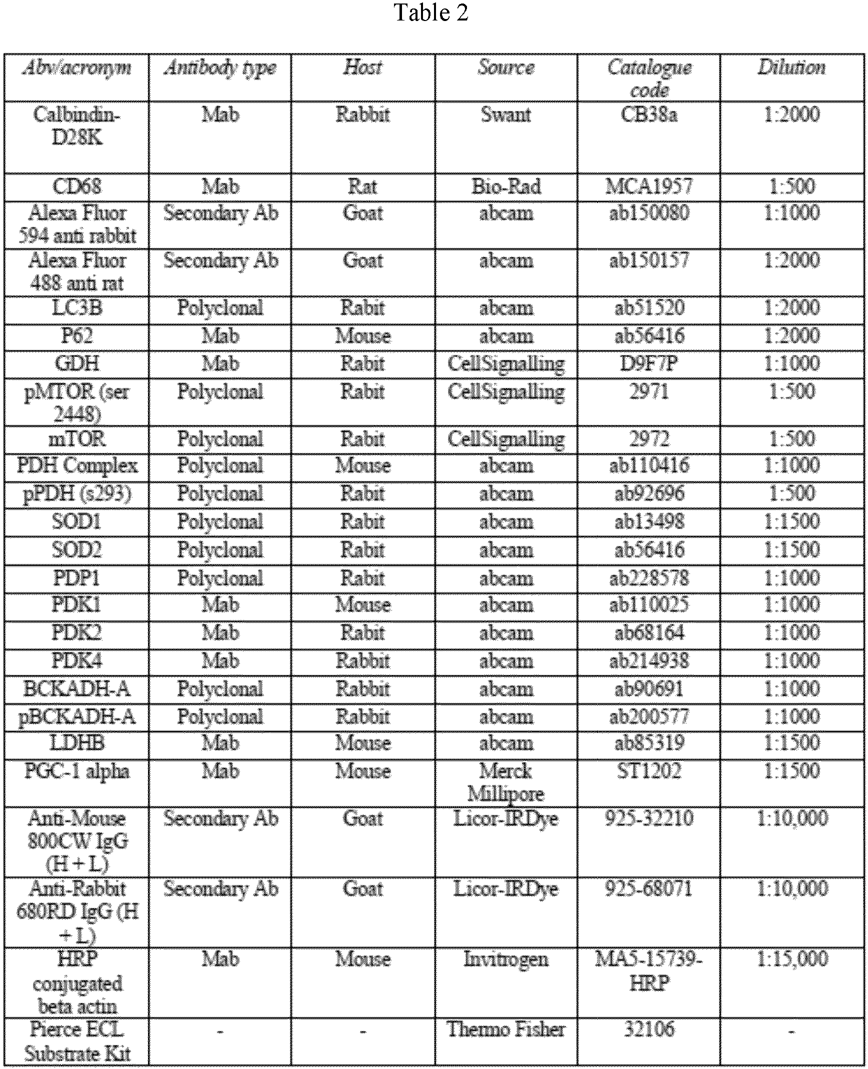

### Acetyl-leucine

ADLL and enantiomers (ADLL: Molekula #73891210, ALL: Sigma Aldrich #441511, ADL: Sigma #A0876) were administered as dry admix in powdered mouse diet (PicoLab Rodent Diet 20/5053, LabDiet) at a dose of 0.1 g/kg/day for *in vivo* studies. Treatment groups for *in vivo* study consisted of at least 5 mice per group. *In vitro* studies were conducted with each compound dissolved in PBS to generate a 50 mM stock solution and diluted to a 1 mM working concentration in media (DMEM High Glucose-Gibco #10566016).

### Miglustat

Miglustat (Monsanto/Oxford GlycoSciences) was administered as a dry solid mixed with powdered chow (600 mg/kg/day) (see above) alone or in combination with ADLL.

### Mouse behavioural analysis.

The weight and activity of each mouse was recorded weekly until reaching the humane endpoint (defined as a loss of 1g body weight within 24 h). Gait analysis was performed using CatWalk 10.5 system (Noldus) according to the manufacturer's instructions and five runs were recorded per animal at each time point. The camera was set to 40 cm below the walkway, the walkway was approximately 4cm wide, and detection settings set to camera gain 14.05 and green intensity 0.12. Motor function was measured using an accelerating NG Rota Rod for mice (Ugo Basile) starting from 1 rpm to 10 rpm, accelerating every 30 seconds by one rpm. Bar crossing experiments on Sandhoff animals were conducted to measure motor strength and coordination. The apparatus used was modified from published studies. Briefly, a metal bar (1.2 mm width and 26 cm in length) was suspended horizontally between two wooden supports 30 cm high over a cushioned surface and animals were allowed to grasp the center of the bar with forepaws only, the tail was released, and the clock was started. Latency to cross or fall from the bar was scored, with 120 sec, maximum, to termination of each trial. A score was given to each animal between +120 and -120. If crossing time was greater than 0, then score = 120- CT; if falling time was greater than 0, the score = FT-120.

### Sample preparation

Biochemical and immunohistochemical analyses. Mice were saline perfused under terminal anaesthesia. Tissues for biochemical analysis were snap-frozen on ice-cold isopentane. For immunofluorescent staining, tissues were perfused with 4% PFA followed by PBS and kept in 4% PFA for 24h and stored in PBS containing 20% sucrose longer term. Brains were cut parasagitally (20 micron sections) and stored in 30% ethylene glycol, 30% glycerol 0.1 M sodium phosphate buffer (pH 7.4) at -20°C. Biochemical analysis was performed on water-homogenised tissues (50 mg/ml) and protein content was determined using a BCA protein assay (Thermo Fisher #23227) according to the manufacturer's instructions.

Western blot analyses. Western blot analysis was carried out by homogenising half cerebellums (ranging from 15 to 30 mg wet weight) in RIPA buffer (Pierce RIPA Buffer, Thermo Fischer Scientific-89900) with protease/phosphatase inhibitor cocktail (Halt^{™} Protease and Phosphatase Inhibitor Cocktail-100X, Cat No: 78440) to achieve 50 mg/ml w/v. Samples then incubated for 30 minutes on ice. They were centrifuged at 13,000 g at 4°C and supernatants were retained for BCA protein assay and western blotting.

ADP and ATP extraction. ADP and ATP were extracted with Phenol-TE from tissues according to published methods. Briefly, freshly prepared tissues were homogenized with 3.0 mL of ice-cold phenol-TE (Sigma-77607) and processed with three cycles of 30-s homogenization and 30-s cooling. One mL of homogenate was transferred into tubes containing 200 uL of chloroform and 150 uL of de-ionized water. The homogenate was thoroughly shaken for 20s and centrifuged at 10,000 × g for 5 min at 4°C (standardise all temps to this format). The supernatant was diluted 1000-fold with deionized water, the diluted extract was used for ADP/ATP analyses.

NAD and NADH extraction. NAD and NADH extractions were performed with 20 mg fresh tissue that was PBS washed on wet ice. Tissues were homogenised with a Dounce homogeniser with 400 uL of NADH/NAD extraction buffer (Abcam NAD/NADH Assay kit-ab65348), followed by centrifugation for 5 min at 4°C at 13.000g. Supernatants were collected into a new tube and filtered through 10kD Spin column (Abcam, #ab93349) at 10,000 g at 4°C until the majority of the liquid had passed through (approximately 20 min).

### Sample analysis

Sphingoid Base Measurements: Sphingoid bases (sphingosine and sphinganine) were extracted from 100 uL tissue homogenates in 500 uL of chloroform: methanol (1:2v/v) followed by sonication for 10 minutes at room temperature. Subsequently, 1 M 500-uL sodium chloride, 500 uL chloroform, and 3 M 100 uL sodium hydroxide were added to the samples, and vortexed every five minutes for 15 minutes at room temperature. Homogenates were centrifuged (13,000 g for 10 minutes) and the lower organic phase retained. Sphingoid bases were purified from the samples by pre-equilibrating SPA-NH2 columns with 2 x 1 mL chloroform followed by sample elution with 3x300 ul acetone. The samples were dried under nitrogen. Lipids were resuspended in 50 uL pre-warmed ethanol (37°C) and 50 uL OPA labelling solution (1 mg OPA/20 uL Ethanol/1 uL 2-mercaptoethanol; dilution 1:2000 in 3% boric acid pH 10.5) was added. Samples were kept at room temperature in the dark for 20 minutes and vortexed every 10 minutes. Samples were buffered with 100 uL methanol: 5 mM Tris pH 7 (9:1) and centrifuged at 5,000 g for 2 minutes. Supernatants (150 uL) were loaded onto a reverse phase HPLC. Chromatography was carried out using a mobile phase of 85% acetonitrile/15% H₂O at a flow rate of 1.0 ml/min. The orthophthaldehyde-labelled derivatives were monitored at an excitation wavelength of 340 nm and an emission wavelength of 450 nm. Quantification of trace peak area was carried out using EZChrom Elite software v3.2.1.

Glycosphingolipid measurements: Glycosphingolipids were extracted and measured by NP-HPLC according to published methods. Tissue homogenates in chloroform/methanol (C:M) (1:2 v/v) overnight at 4°C. The mixture was centrifuged (3000 rpm/10 min) and 1 mL chloroform and 1 mL PBS were added to the supernatant and centrifuged (3000 rpm/10 min). The lower phase was dried under N₂, resuspended in 50 uL C:M 1:3 v/v and combined with the upper phase. Subsequently, GSLs were recovered using C18 Isolute columns (100 mg) (Biotage) pre-equilibrated with 4x1 mL MeOH and 2x1 mL H₂O. Samples were washed 3x1 mL H₂O and eluted with 1 mL C:M 98:2, 2x1 mL C:M 1:3, 1 mL MeOH. The column eluate was dried under N₂, resuspended in 100 uL C:M 2:1, dried again under N2 and resuspended in ceramide glycanase (CGase) buffer (50 mM sodium acetate pH 5.5, 1 mg/mL sodium taurodeoxycholate). 50 mU CGase was added, and samples incubated at 37°C overnight. Released oligosaccharides were anthranilic acid (2-AA) labelled and purified by mixing with 1 mL Acetonitrile: H₂O 97:3 and added to Discovery DPA-6S columns (SUPELCO, # 52625-U) pre-equilibrated with 1 mL acetonitrile, 2x1 mL H₂O, and 2x1 mL acetonitrile. Columns were washed with 2x1 mL Acetonitrile: H₂O 95:5 v/v, and eluted in 2x 0.75 mL H₂O. Samples were loaded 30:70 H₂O: MeCN (v/v) for normal phase HPLC. Solvent A was acetonitrile, solvent B was H₂0, and solvent C was 100 mM NH₄OH (pH 3.85) in H₂0. Separation was carried out at 30°C using Waters Alliance 2695 separation module, excitation 360, emission 425 using Waters 2475 Fluorescence detector.

Cholesterol measurements: Cholesterol was measured with the Amplex Red kit (Molecular Probes) according to the manufacturer's instructions. Briefly, cell and tissue homogenates in 100 uL mQ water were Folch extracted and dried down under nitrogen. The pellets containing cholesterol were resuspended in 1X Reaction Buffer, and 50 uL was loaded for each sample in a flat bottom 96-well plate. The reaction was initiated by adding Working Solution per sample (25% Amplex^{®} Red, 2U/mL HRP stock solution, 2 U/mL cholesterol oxidase stock solution, 0.2 U/mL cholesterol esterase stock solution in 1X Reaction Buffer). Samples were incubated at 37°C for 30 minutes and the fluorescence was measured in a microplate reader (Optima, BMG Labtech) using excitation in the range of 530-560 nm and emission detection at ~590 nm.

Lysotracker Green and Propidium Iodide Staining of CHO cells: *In vitro* FACS experiments were performed in order to measure relative acidic compartment volume staining live cells with LysoTracker^{™} Green DND-26 (Thermo Fisher-L7526) at 250 nM for 10 minutes in PBS at RT, centrifuged at 1200rpm for 10 min and cells resuspended in FACS buffer (PBS, 1% BSA, 0.1% NaN3 sodium azide). Cells were stained with propidium iodide (20 nM) (Invitrogen-P3566) immediately prior to analysis on the FACS for dead cell discrimination. FACS analysis was performed on 10,000 recorded cells using FACS Canto (Becton Dickinson, (BD)) with BD software.

Filipin Staining: Free cellular cholesterol in CHO cells was visualized with Filipin (from *Streptomyces filipinensis* (Sigma)). Cells were fixed with 4% paraformaldehyde, washed 3 x PBS and incubated with 1.5 mg/mL glycine for 10 min to quench auto-fluorescence. Cells were incubated with filipin (0.05 mg/mL in PBS/10% FBS/0.2% Triton x100) for 2 hours, washed 3xPBS and visualized by Leica-SP8 confocal microscope.

Immunohistochemistry: Free-floating brain sections in glycerol/ethylene were rinsed 3 times with PBS and blocked with 2% goat serum in 0.3% PBS Triton at room temperature. Sections were stained with primary antibodies; rabbit Calbindin (1:2000) and rat CD68 (1:500) in 2% goat serum in 0.3% PBS for 16 hours at 4°C, followed by washing with 0.3% PBS Triton, then 3 times with PBS. Secondary antibody staining (Alexa Fluor 597 Goat anti rabbit Far Red, Alexa Fluor 488 Goat anti rat) was conducted at room temperature for 2 hours. The antibodies used are summarised in Table 2. Samples were washed once with 0.3% PBS Triton, 3 times with PBS and manually mounted on slides. Slides left on the bench overnight to dry the sections followed by application of ProLong Gold (Invitrogen, #P36930).

Western Blotting: Homogenates of cerebellum (50 mg/ml) were stored with 1% Igepal CA, 0.5% sodium deoxycholate, 0.1% SDS, and 1% protease-phosphatase inhibitor cocktail. Samples were resolved using 4-12% SDS Tris-Glycine (Biorad) gel electrophoresis and transferred to low fluorescent PVDF membrane (Biorad Immunblot Low Fluorescence PVDF paper-#1620262) using a semidry transfer apparatus (Trans-Blot Turbo Transfer System (Biorad #1704150)). The antibodies used are summarised in Table 2.

Image Acquisition and Quantification: Brain sections and CHO cells imaging was performed with a Leica-SP8 confocal microscope in tile imaging mode. Western blot data acquisition was conducted with LiCOR Odyssey Infrared Imaging system model no 9120. Mean fluorescence values, cerebellum diameters and areas, cell quantifications were calculated with Fuji Version 1.0 software.

ADP/ATP measurements: ADP/ATP measurements were made using a kit (Sigma Aldrich, MAK135) according to the manufacturer's instructions. The ADP/ATP ratio was calculated with the equation: (RLU-C-RLUB)/RLU-A.

NAD and NADH measurements: NAD, NADH and total NAD (NADt) (NAD+NADH = NADt) were measured with the NAD/NADH assay kit (Abcam-ab65348). Fresh samples were processed for ADP ATP extraction. Each sample was divided into 2: 1) Normal samples 2) Decomposed samples. Decomposition of each sample was achieved by heating samples at 60°C for 30 minutes on a heating block. 30 uL extracts were loaded into flat bottom clear 96 well plates, each sample comprising 3 wells: 1) Background 2) NADt 3) Decomposed samples (contains only NADH). Background reaction mix (60 uL) was loaded into "background wells", and Reaction Mix was loaded into "NADt" and "Decomposed sample" wells. The plate was incubated at room temperature for 5 minutes, and 6 uL NADH developer solution was added to the "Decomposed sample" wells. Absorbance readings were taken at 30 min intervals over 3h at 450 nm.

Demographics and statistical analysis of a clinical observational study of adult NPC patients treated with ADLL: The cohort comprised 3 females and 10 males. Consent from all participants was obtained. Twelve of the NPC patients were on miglustat therapy (minimum exposure 2.65, 1^{st} quartile 3.44, median 4.92, mean 4.87, 3^{rd} quartile 5.80 and maximum 7.98 years). The thirteen NPC1 patients participated in an observational study treated with 5g/day acetyl-DL-leucine (Tanganil^{™}). Three patients had four prospective clinical severity score measurements and ten patients had five measurements taken during this observational study (NIH, CSS). Retrospective data were used to calculate rates of disease progression prior to recruitment to this study. The mean age at the start of acetyl-DL-leucine (Tanganil^{™}) treatment was 26.59 years. The minimum age was 15.44, 1^{st} quartile 20.97, median 26.98 and third quartile 30.79 with maximum age 37.37 years. The time in the observational treatment study was a mean of 0.53, 1^{st} quartile 0.81, median 1, mean 0.95, third quartile 1.13 and maximum 1.21 years.

Observational studies in GM2 gangliosidosis Patients: In a prospective observational case series in three patients with genetically proven GM2 gangliosidoses (2 patients with Tay-Sachs disease, 1 patient with Sandhoff disease) the symptomatic effects of ADLL was evaluated. Patients were treated with 0.1 g per kg body weight per day, for at least four weeks and the clinical effects determined using the Scale for the Assessment and Severity of Ataxia (SARA), the 8-meter walk test (8MWT) and the Montreal Cognitive Assessment (MoCA). Consent from all participants was obtained.

### Patient 1:

A 28-yearsold male patient with a genetically confirmed diagnosis of Tay-Sachs disease presented with dysarthrophonia, tremor, ataxia of stance and gait, paraparesis, and muscular atrophy. Prior to treatment, the patient required the support of both a caregiver on one side and a wall on the other in order to walk down a corridor. The patient was receiving sodium-valproate (500 mg/d), lithium (450 mg/d), and risperidone (3 mg/d) (medications for clinically diagnosed bipolar disorder), and continued to do so during treatment with N-Acetyl-DL-Leucine. The patient was also receiving physiotherapy, ergotherapy, and logotherapy twice a week.

### Patient 2:

A 32-year-old female patient with a genetically confirmed diagnosis of Tay-Sachs disease presented with ataxia of stance and gait, fine motor impairment, paraparesis of lower extremities and muscular atrophy. She was receiving physiotherapy, ergotherapy and logotherapy and continued to do so during treatment with N-Acetyl-DL-Leucine.

### Patient 3:

An 8-year-old male patient suffering from Sandhoff disease, with muscle hypotrophy, epileptic cramps (tonic-clonic, about 10 seconds, self-limiting), disordered ocular movement and anarthria. His daily activities were very limited, being unable to eat, wash, or dress himself. He was receiving levetiracetam (1 g/d), lamotrigine (100 mg/d), omeprazole (20 mg/d), and miglustat (300 mg/d), in addition to physiotherapy, ergotherapy and logotherapy, and continued to do so during treatment with N-Acetyl-DL-Leucine.

### Statistical Analyses: murine studies

Differences between groups were identified either by 1-way or 2-way analyses of variances (ANOVA); comparisons of groups were made after 2-way ANOVAs with Fisher's LSD test. Statistical tests were performed using GraphPad Prism 6 software (La Jolla, CA).

### EXAMPLE 1

### ADLL, ALL and ADL administered during the symptomatic phase of disease improves ataxia in Npc1^{-/-} mice

*Npc1*^{*-*/*-*} mice have a 10-12-week life span, with onset of symptoms (gait abnormalities, tremor and weight loss) beginning at 6-7 weeks of age. Williams et al., Neurobiol. Dis. 67:9-17 (2014). ADLL, ALL, and ADL were administered to *Npc1*^{*-*/*-*} mice in the diet (0.1g/kg/day), a dose similar to that used in observational clinical studies. Bremova, T. et al, Neurology 85:1368-1375 (2015). Untreated 9-week-old *Npc1*^{*-*/*-*} mice exhibit statistically significant (p <0.0001) ataxia that presents as a sigmoidal gait, relative to wild-type mice, whereas 9-week-old *Npc1*^{*-*/*-*} mice treated with ADLL, ALL or ADL from 8-weeks of age (1 week of treatment) all displayed significantly reduced ataxia as determined by measuring lateral displacement from a straight trajectory in an automated gait analysis system (Fig. 1 and Fig. 2) *(p* <0.0001, all treatments).

### EXAMPLE 2

### Pre-symptomatic treatment with ADLL, ALL and ADL improves ataxia in Npc1^{-/-}mice

*Npc1*^{*-*/*-*} mice were treated pre-symptomatically from weaning (3 weeks of age) and assessed at 9 weeks of age (6 weeks of treatment). ADLL, ALL, and ADL significantly reduced the magnitude of ataxia (Fig. 3 and Fig. 4) with the magnitude comparable to the effects observed with one week of treatment.

### EXAMPLE 3

### Pre-symptomatic treatment with ADLL and ALL improves gait abnormalities, motor function, and modestly extends survival in Npc1^{-/-}mice

Npc1^{-/-} mice develop motor dysfunction (measured by Rotarod) and paw placement abnormalities when ambulatory (measured with CatWalk). Npc1^{-/-} mice were treated pre-symptomatically from weaning (3 weeks of age) and assessed the mice at 8 weeks of age. Untreated Npc1^{-/-} mice had a significantly increased hind paw duty cycle (Fig. 5) (percentage of hind paws in contact with the platform relative to the elapsed time between two consecutive contacts of the same paw) and a reduced hind paw stand mean (average duration of paw in contact with the platform) relative to wild-type mice. (Fig. 6). Pre-symptomatic ADLL and ALL treatment of Npc1^{-/-} mice resulted in a significantly reduced (functional improvement) duty cycle of hind paws (ADLL: p = 0.0005, ALL: p <0.0001) (Fig. 5) and hind paw stand mean (indicative of functional improvement) (ADLL: p = 0.0179, ALL: p = 0.0014) relative to untreated Npc1^{-/-} mice (Fig. 6). Rotarod performance was significantly impaired in untreated Npc1^{-/-} mice relative to wild-type littermates (p <0.0001). Treatment with ADLL (p = 0.0088) and ALL (p = 0.0070) resulted in a significant improvement in Rotarod performance by Npc1^{-/-} mice (Fig. 7). ADL treatment had no significant benefit on Npc1^{-/-} mouse motor function, as assessed using Catwalk (p <0.95) or Rotarod (p = 0.2218), suggesting that the D enantiomer has no benefit on these specific functional parameters. Relative to untreated Npc1^{-/-} mice, the life span of animals treated from weaning was significantly increased by 8 days (9.1%) with ALL treatment (p = 0.0334), 4 days with ADLL (p = 0.0305) (4.5%) and was not changed with ADL (p = 0.6908) (Fig. 8).

### EXAMPLE 4

### Improved neuropathology and lipid storage in Npc1^{-/-} mouse brain in a stereo-selective manner

In light of the benefits of ADLL and ALL on gait, motor function and survival when treatment was initiated in the pre-symptomatic period, the impact of ALs on lipid storage in Npc1^{-/-} mice was evaluated. Since NPC1 disease is characterized by the accumulation of sphingoid bases (sphingosine and sphinganine), cholesterol, sphingomyelin, free fatty acids and glycosphingolipids (GSLs), the impact of ALs administered to Npc1^{-/-} mice from 3 weeks of age measuring lipid storage in brain was measured. At 59 days of age Npc1^{-/-} mice exhibited increased lipid levels relative to wild-type (Figs. 9, 10A, 10B, 11, 12A, and 12b). In order to evaluate the differential impact of the AL analogues in the CNS, the cerebellum and the forebrain (referred to as brain in the figures) were measured separately. Total GSLs in the forebrain were not significantly altered by any of the AL treatments (Fig. 27A), whilst ALL selectively reduced GM1a (20.1%; p = 0.0018) and GM2 (19.6%; p = 0.0222) (Fig. 9). Interestingly, although sphingosine levels were not significantly affected, ADLL and ADL reduced sphinganine levels by 13.5% (p = 0.0456) and 18.2% (p = 0.0111) respectively (Fig. 10A and 10B). Analysis of the cerebellum confirmed there was no significant difference in total GSLs relative to untreated Npc1^{-/-} mice with any of the AL analogues tested (Fig. 27B). However, ADLL and ALL treatment significantly lowered levels of specific GSLs including GA2 (ADLL: 21.3%, p = 0.0102; ALL: 23.8%, p = 0.0042), whereas a significant reduction in GA1 was only observed with ALL treatment (23.5%, p = 0.0254) (Fig. 11). Comparable to the results for the forebrain, sphingosine was unchanged in the cerebellum, but sphinganine was significantly reduced following ADL treatment (42.3%, p = 0.0074) (Fig. 12A and Fig. 12B).

### EXAMPLE 5

### ADLL ameliorates neurodegeneration and ALL reduces neuroinflammation in the cerebellum

*Npc1*^{*-*/*-*} mice exhibit progressive neurodegeneration, with cerebellar Purkinje cell loss progressing from anterior to posterior lobes, accompanied by microglial activation. ADLL treatment increased Purkinje cell survival at 59 days of age relative to untreated *Npc1*^{*-*/*-*} littermates: 133% more Purkinje cells were present in lobules I and II (*p* = 0.0027), and 402% more Purkinje cells in lobule III *(p* = 0.0108) (Fig. 13). Other treatments did not significantly improve Purkinje cell survival in any cerebellar lobules (*Npc1*^{*-*/*-*}ALL Lobule I-IIp = 0.107, Lobule III *p* = 0.157, Lobule IV-V *p* = 0.533; *Npc1*^{*-*/*-*} ADL Lobule I-II *p* = 0.60, Lobule III *p* = 0.11, Lobule IV-V *p* = 0.766, compared to *Npc1*^{*-*/*-*} UT) (Fig. 13). In addition, only ALL treatment significantly reduced (by 20%, *p* = 0.0177) the frequency of CD68 positive activated microglia (Fig. 14) while other treatments did not have a significant impact (p = 0.1353 for *Npc1*^{*-*/*-*} ADLL, p = 0.0553 for *Npc1*^{*-*/*-*}ADL compared to *Npc1*^{*-*/*-*} UT). ADL did not mediate any long term, neuroprotective effects.

### EXAMPLE 6

### AL treatments alleviate lipid storage in non-neuronal tissues and cells

Liver GSL levels in the Npc1^{-/-} mice were significantly reduced in Npc1^{-/-} mice treated with ADLL (26.9%, p = 0.03), ADL (26.9%, p = 0.0253) and 45.6% for ALL (p = 0.0003) (Fig. 27C). Quantification of the major GSL species confirmed that levels of GM2Gc (the most abundant GSL in the mouse liver) was decreased significantly with all AL analogues tested (26.7% ADLL: p = 0.0002, 45% ALL: p <0.0001, 29% ADL: p <0.0001), while GM3Gc levels were only reduced significantly by ALL (54.8%: p = 0.0314) relative to untreated Npc1^{-/-} mice (Fig. 15A). All analogues tested caused significant and comparable levels of reduction of sphingosine (the catabolic break down product of ceramide) (ADLL 29.5%, p = 0.0233; ALL 33.2%, p = 0.0148; ADL 33.6% p = 0.0103) and its de novo precursor sphinganine (ADLL 32.5% p = 0.0365; ALL 37.4% p = 0.0189; ADL 38.5% p = 0.0163) (Fig. 15B and Fig. 15C). Total free cholesterol in Npc1^{-/-} liver was also significantly decreased after treatment with ADLL, ALL or ADL (ADLL 18.2% p = 0.0346; ALL 23.4% p = 0.0091; ADL 16.2 % p = 0.0210) (Fig. 15D). To explore whether other non-neuronal cell types were corrected by ALs the lysosomal volume as a surrogate for storage in Npc1^{-/-} Chinese Hamster Ovary (CHO) cells was evaluated. Treatment with 1 mM ADLL and ALL reduced relative lysosomal volume after 24h (ADLL 16.9%, p = 0.0137; ALL 16%, p = 0.0177); however, this beneficial effect was seen with all three acetyl leucine treatments after 72h of treatment (ADLL 24% p = 0.0026; ALL 22.8% p = 0.0036; ADL 22.3 % p = 0.0042) (Fig. 16). After 72 hours AL treatment sphingosine levels were significantly reduced by 22% with ADLL (p = 0.0009), 29% with ALL (p <0.0001) and 14% with ADL (p = 0.0149) (Fig. 17). Total GSL levels were significantly reduced with all treatments to a comparable extent (26% with ADLL p = 0.0007; 25% with ALL p = 0.0010; and 22% with ADL p = 0.0024) (Fig. 17). Likewise, cholesterol content was significantly reduced (18% with ADLL p = 0.0043; 13% with ALL p = 0.0263; and 14% with ADL p = 0.0212) (Fig. 17).

### EXAMPLE 7

### ADLL synergises with miglustat in Npc1^{-/-} mice

*Npc1*^{*-*/*-*} mice were treated with ADLL in combination with the standard of care drug miglustat, resulting in a statistically significant longer life span than animals on monotherapy (*Npc1*^{*-*/*-*} miglustat vs *Npc1*^{*-*/*-*} miglustat & ADLL *p* = 0.0016; *Npc1*^{*-*/*-*} ADLL vs *Npc1*^{*-*/*-*} miglustat & ADLL *p* = 0.0063; median survival; *Npc1*^{*-*/*-*}UT: 87 days, *Npc1*^{*-*/*-*} / ADLL: 91 days; *Npc1*^{*-*/*-*} miglustat: 117 days, *Npc1*^{*-*/*-*} miglustat & ADLL: 138 days) (Fig. 18). Miglustat is known to cause weight loss through appetite suppression. When ADLL was used in combination with miglustat it prevented the weight loss associated with miglustat treatment (Fig. 19) but not the weight loss resulting from progression of the disease. Combination therapy significantly increased Rotarod test performance (8 weeks: *p* = 0.0007; 10,12 weeks *p <0.0001* versus untreated *Npc1*^{*-*/*-*}), with combination therapy associated with increased benefit relative to mice receiving either miglustat (10 weeks: p = 0.0299; 12 weeks: *p* = 0.0232) or ADLL monotherapy (10,12 weeks: *p* <*0.0001*) (Fig. 20). Gait analysis at 10 weeks of age demonstrated that hind Duty Cycle percentages were significantly improved with combination therapy (*p* = 0.0028) whereas miglustat monotherapy showed no benefit (Fig. 21). Stand mean significantly improved in mice treated either with combination therapy *(p* = 0.0250) or miglustat monotherapy (*p* = 0.0196) (Fig. 22).

### EXAMPLE 8

### Neuroprotective effects of ADLL in observational clinical studies in NPC patients

Unexpected beneficial effects of ALs in *Npc1*^{*-*/*-*} mice were discovered. Whether neuroprotective effects also occur in NPC patients treated with ADLL enrolled in an observational clinical study was investigated. Total clinical severity scores (maximum score 50, with higher values equating to increasing levels of disability) were plotted prior to initiation of treatment with Tanganil^{™}, including available retrospective data (Fig. 23). All 13 patients had positive slopes of disease progression before ADLL treatment (1.8 severity units/year was the average slope). Following initiation of ADLL treatment the average slope was -1.78 (3 patients had no slope, 10 had negative slopes) equating to a significant reduction in disease progression and improvement in the majority of patients (One-sided sign test *p* = 0.0002). The data were also computed as Annual Severity Increment Scores that measures the rate of disease progression and a mean of -9.1% / year *(p* <0.001) in ASIS scores was observed. When the treated patient data were analysed by neurological subdomain the majority of patients improved or stabilised on treatment, irrespective of the neurological subdomain measured (Table 1).

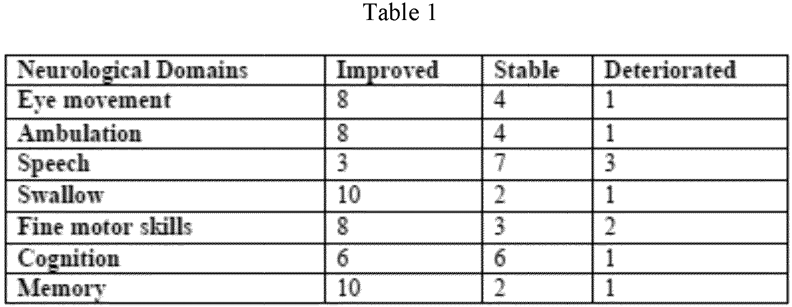

### EXAMPLE 9

### ADLL shows benefit in Sandhoff mice and GM2 gangliosidosis patients

The Sandhoff (Hexb-/-) mouse model is pre-symptomatic up to 6-8 weeks of age. Subsequently, these mice develop tremor and their motor function begins to decline. By the later stages of the disease (12-15 weeks) they are inactive and are unable to complete motor function tests such as bar crossing. *Hexb*^{*-*/*-*} animals were treated from weaning with ADLL (0.1 mg/kg/day, the same dose used for treatment of *Npc1*^{*-*/*-*} mice). Extended survival, with a median increase in lifespan of 8 days (*p* = 0.0157) (Fig. 24) was observed. Bar-crossing test performance also significantly improved (12 weeks: *p* = 0.0343; 13 weeks: p = 0.0058), which is indicative of delayed deterioration in motor function (Fig. 25).

These findings in Sandhoff mice were extended to observational clinical studies in three patients with a confirmed GM2 gangliosidosis diagnosis (2 Tay-Sachs and 1 Sandhoff disease) treated with ADLL. A mean improvement of the Scale for Assessment and Rating of Ataxia (SARA) by 20.3%, the 8-M-Walking-Test (8MWT) by 17.8% and the Montreal Cognitive Assessment (MoCA) by 42% was found as shown in the Fig. 26. All patients and caregivers also reported a subjective improvement and are still on treatment at the same dosage.

### References

Neuzil, E., Ravaine, S. & Cousse, H. La N-acetyl-DL-leucine, medicament symptomatique des etats vertigineux. Bull. Soc. Pharm. Bordeaux 141, 15-38 (2002).

Vibert, N. & Vidal, P.-P. In vitro effects of acetyl- dl -leucine (tanganil®) on central vestibular neurons and vestibulo-ocular networks of the guinea-pig. Eur. J. Neurosci. 13, 735-748 (2001).

Strupp, M. et al. Effects of acetyl-dl-leucine in patients with cerebellar ataxia: A case series. J. Neurol. 260, 2556-2561 (2013).

Schniepp, R. et al. Acetyl-DL-leucine improves gait variability in patients with cerebellar ataxia-a case series. Cerebellum & ataxias 3, 8 (2016).

Bremova, T. et al. Acetyl-DL-leucine in Niemann-Pick type C: A case series. Neurology 85, 1368-1375 (2015).

Platt, F. & Strupp, M. An anecdotal report by an Oxford basic neuroscientist: effects of acetyl-dl-leucine on cognitive function and mobility in the elderly. Journal of Neurology 263, 1239-1240 (2016).

Günther, L. et al. N-Acetyl-L-Leucine accelerates vestibular compensation after unilateral labyrinthectomy by action in the cerebellum and thalamus. PLoS One 10, 1-18 (2015).

Tighilet, B., Leonard, J., Bernard-Demanze, L. & Lacour, M. Comparative analysis of pharmacological treatments with N-acetyl-dl-leucine (Tanganil) and its two isomers (N-acetyl-L-leucine and N-acetyl-D-leucine) on vestibular compensation: Behavioral investigation in the cat. Eur. J. Pharmacol. 769, 342-349 (2015).

Platt, F. M. Emptying the stores: Lysosomal diseases and therapeutic strategies. Nature Reviews Drug Discovery (2018). doi:10.1038/nrd.2017.214

Cortina-Borja, M. et al. Annual severity increment score as a tool for stratifying patients with Niemann-Pick disease type C and for recruitment to clinical trials. Orphanet J. Rare Dis. 13, 1-16 (2018).

Williams, I. M. et al. Neurobiology of Disease Improved neuroprotection using miglustat , curcumin and ibuprofen as a triple combination therapy in Niemann - Pick disease type C1 mice. Neurobiol. Dis. 67, 9-17 (2014).

Kirkegaard, T. et al. Heat shock protein-based therapy as a potential candidate for treating the sphingolipidoses. Sci. Transl. Med. 8, (2016).

Lloyd-Evans, E. & Platt, F. M. Lipids on trial: The search for the offending metabolite in Niemann-Pick type C disease. Traffic 11, 419-428 (2010).

Te Vruchte, D. et al. Relative acidic compartment volume as a lysosomal storage disorder-associated biomarker. J. Clin. Invest. 124, 1320-1328 (2014).

Priestman, D. A., Van Der Spoel, A. C., Butters, T. D., Dwek, R. A. & Platt, F. M. N-butyldeoxynojirimycin causes weight loss as a result of appetite suppression in lean and obese mice. Diabetes, Obes. Metab. (2008). doi:10.1111/j.1463-1326.2006.00701.x

Kimball, S. R., Shantz, L. M., Horetsky, R. L. & Jefferson, L. S. Leucine regulates translation of specific mRNAs in L6 myoblasts through mTOR-mediated changes in availability of eIF4E and phosphorylation of ribosomal protein S6. J. Biol. Chem. (1999). doi:10.1074/jbc.274.17.11647

Klionsky, D. J. & Emr, S. D. Autophagy as a regulated pathway of cellular degradation. Science 290, 1717-1721 (2000).

Yanagisawa, H. et al. L-leucine and SPNS1 coordinately ameliorate dysfunction of autophagy in mouse and human Niemann-Pick type C disease. Sci. Rep. 7, 1-9 (2017).

Boland, B. et al. Macroautophagy is not directly involved in the metabolism of amyloid precursor protein. J. Biol. Chem. 285, 37415-37426 (2010).

Pankiv, S. et al. p62/SQSTM1 binds directly to Atg8/LC3 to facilitate degradation of ubiquitinated protein aggregates by autophagy*[S]. J. Biol. Chem. (2007). doi:10.1074/jbc.M702824200

Harris, R. a, Joshi, M., Jeoung, N. H. & Obayashi, M. Overview of the molecular and biochemical basis of branched-chain amino acid catabolism. J. Nutr. 135, 1527S-30S (2005).

Yudkoff, M. Brain metabolism of branched-chain amino acids. Glia 21, 92-8 (1997).

Kennedy, B. E., Hundert, A. S., Goguen, D., Weaver, I. C. G. & Karten, B. Presymptomatic Alterations in Amino Acid Metabolism and DNA Methylation in the Cerebellum of a Murine Model of Niemann-Pick Type C Disease. Am. J. Pathol. 1-18 (2016). doi:10.1016/j.ajpath.2016.02.012

Kato, M. et al. Structural Basis for Inactivation of the Human Pyruvate Dehydrogenase Complex by Phosphorylation: Role of Disordered Phosphorylation Loops. Structure 16, 1849-1859 (2008).

Huiyun, L. & Walter, W. F. PGC-1alpha: a key regulator of energy metabolism. Adv. Physiol. Educ. 30, 145-151 (2006).

Jha, M. K., Jeon, S. & Suk, K. Pyruvate Dehydrogenase Kinases in the Nervous System: Their Principal Functions in Neuronal-glial Metabolic Interaction and Neuro-metabolic Disorders. Curr Neuropharmacol 10, 393-403 (2012).

Yanjanin, N. M. et al. Linear clinical progression, independent of age of onset, in Niemann-Pick disease, type C. Am. J. Med. Genet. Part B Neuropsychiatr. Genet. (2010). doi:10.1002/ajmg.b.30969

Jeyakumar, M. et al. Delayed symptom onset and increased life expectancy in Sandhoff disease mice treated with N-butyldeoxynojirimycin. Proc. Natl. Acad. Sci. U. S. A. 96, 6388-93 (1999).

Nagamori, S. et al. Structure-activity relations of leucine derivatives reveal critical moieties for cellular uptake and activation of mTORC1-mediated signaling. Amino Acids 48, 1045-1058 (2016).

Harris, A., Paxton, R., Powell, S. M. & Gillim, S. E. Physiological Covalent Regulation of Rat Liver Branched-Chain a-Ketoacid Dehydrogenase. Arch. Biochem. Biophys. 243, 542-555 (1985).

Son, S. M. et al. Leucine Signals to mTORC1 via Its Metabolite Acetyl-Coenzyme A. Cell Metab. 0, 1-10 (2018).

Kennedy, B. E. et al. Pre-symptomatic activation of antioxidant responses and alterations in glucose and pyruvate metabolism in niemann-pick type C1-deficient murine brain. PLoS One 8, (2013).

Ruiz-Rodado, V. et al. 1H NMR-Linked Metabolomics Analysis of Liver from a Mouse Model of NP-C1 Disease. J. Proteome Res. 15, 3511-3527 (2016).

Liana Roberts Stein & Imai, S. The dynamic regulation of NAD metabolism in mitochondria. Trends Endocrinol Metab. 23, 420-428 (2012).

Murphy, M. P. & Hartley, R. C. Mitochondria as a therapeutic target for common pathologies. Nat. Rev. Drug Discov. 1, (2018).

Pliss, L. et al. Cerebral Developmental Abnormalities in a Mouse with Systemic Pyruvate Dehydrogenase Deficiency. PLoS One 8, 1-14 (2013).

Platt, F. M., Boland, B. & van der Spoel, A. C. The cell biology of disease: lysosomal storage disorders: the cellular impact of lysosomal dysfunction. J. Cell Biol. 199, 723-34 (2012).

Wiederschain, G. Y. The metabolic and molecular bases of inherited disease. Biochemistry (Moscow) 67, 611 (2002).

Sandhoff, K. & Harzer, K. Gangliosides and Gangliosidoses: Principles of Molecular and Metabolic Pathogenesis. J. Neurosci. 33, 10195-10208 (2013).

Pentchev, P. G. et al. A lysosomal storage disorder in mice characterized by a dual deficiency of sphingomyelinase and glucocerebrosidase. Biochim. Biophys. Acta - Lipids Lipid Metab. (1980). doi:10.1016/0005-2760(80)90116-2

Sango, K. et al. Mouse models of Tay-Sachs and Sandhoff diseases differ in neurologic phenotype and ganglioside metabolism. Nat. Genet. (1995). doi:10.1038/ng1095-170

Barclay, L. L., Gibson, G. E. & Blass, J. P. The string test: An early behavioral change in thiamine deficiency. Pharmacol. Biochem. Behav. (1981). doi:10.1016/0091-3057(81)90236-7

Chida, J., Yamane, K., Takei, T. & Kido, H. An efficient extraction method for quantitation of adenosine triphosphate in mammalian tissues and cells. Anal. Chim. Acta 727, 8-12 (2012).

Neville, D. C. A. et al. Analysis of fluorescently labeled glycosphingolipid-derived oligosaccharides following ceramide glycanase digestion and anthranilic acid labeling. Anal. Biochem. (2004). doi:10.1016/j.ab.2004.03.051

All of the features described herein (including any accompanying claims, abstract and drawings), and/or all of the steps of any method so disclosed, may be combined with any of the above aspects in any combination, except combinations where at least some of such features and/or steps are mutually exclusive.

## Claims

1. Acetyl-leucine and miglustat for use in a method of treating a lysosomal storage disease in a subject in need thereof, the method comprising administering a combination of:
(i) a therapeutically effective amount of acetyl-leucine; and
(ii) a therapeutically effective amount of miglustat,
to the subject, wherein subject is naive to treatment with miglustat.

2. The acetyl-leucine and miglustat for use of claim 1, wherein acetyl-leucine and miglustat are administered simultaneously.

3. The acetyl-leucine and miglustat for use of claim 2, wherein acetyl-leucine and miglustat are administered as a single pharmaceutical formulation or as two separate pharmaceutical formulations.

4. The acetyl-leucine and miglustat for use of claim 1, wherein acetyl-leucine and miglustat are administered sequentially.

5. The acetyl-leucine and miglustat for use of claim 4, wherein acetyl-leucine is administered before or after miglustat.

6. The acetyl-leucine and miglustat for use of claim 5, wherein acetyl-leucine and miglustat are administered about 1 minute to about 6 hours apart, preferably wherein acetyl-leucine and miglustat are administered about 1 minute to 3 hours apart, more preferably about 1 minute to 1 hour apart.

7. The acetyl-leucine and miglustat for use of any one of claims 1-6, wherein acetyl-leucine and miglustat are administered orally.

8. The acetyl-leucine and miglustat for use of any one of claims 1-7, wherein acetyl-leucine is administered once, twice, or three times per day.

9. The acetyl-leucine and miglustat for use of any one of claims 1-8, wherein miglustat is administered once, twice, or three times per day.

10. The acetyl-leucine and miglustat for use of any one of claims 1-9, wherein about 3 g to about 15 g of acetyl-leucine is administered per day.

11. The acetyl-leucine and miglustat for use of any one of claims 1-10, wherein about 0.05 g to about 2 g of miglustat is administered per day.

12. The acetyl-leucine and miglustat for use of any one of claims 1-11, wherein acetyl-leucine and miglustat are administered as first-line therapy to treat the lysosomal storage disease.

13. The acetyl-leucine and miglustat for use of any one of claims 1-12, wherein the lysosomal storage disease is Niemann-Pick Disease Type C.

14. The acetyl-leucine and miglustat for use of any one of claims 1-13, wherein the acetyl-leucine is N-acetyl-DL-leucine or N-acetyl-L-leucine.

15. A kit comprising acetyl-leucine and miglustat for use in a method of treating a lysosomal storage disease in a subject, wherein subject is naive to treatment with miglustat, optionally wherein said kit further comprises instructions for administering the acetyl-leucine and miglustat to the subject.

## Patentansprüche

1. Acetyl-Leucin und Miglustat zur Verwendung in einem Verfahren zur Behandlung einer lysosomalen Speicherkrankheit bei einem Patienten mit entsprechendem Behandlungsbedarf, wobei das Verfahren das Verabreichen einer Kombination aus:
(i) einer therapeutisch wirksamen Menge von Acetyl-Leucin; und
(II) einer therapeutisch wirksamen Menge von Miglustat an den Patienten umfasst, wobei der Patient bisher noch nicht mit Miglustat behandelt wurde.

2. Acetyl-Leucin und Miglustat zur Verwendung nach Anspruch 1, wobei Acetyl-Leucin und Miglustat gleichzeitig verabreicht werden.

3. Acetyl-Leucin und Miglustat zur Verwendung nach Anspruch 2, wobei Acetyl-Leucin und Miglustat als einzelne pharmazeutische Formulierung oder als zwei getrennte pharmazeutische Formulierungen verabreicht werden.

4. Acetyl-Leucin und Miglustat zur Verwendung nach Anspruch 1, wobei Acetyl-Leucin und Miglustat nacheinander verabreicht werden.

5. Acetyl-Leucin und Miglustat zur Verwendung nach Anspruch 4, wobei Acetyl-Leucin vor oder nach Miglustat verabreicht wird.

6. Acetyl-Leucin und Miglustat zur Verwendung nach Anspruch 5, wobei Acetyl-Leucin und Miglustat mit einem Abstand von ungefähr 1 Minute bis ungefähr 6 Stunden verabreicht werden, wobei Acetyl-Leucin und Miglustat vorzugsweise mit einem Abstand von ungefähr 1 Minute bis 6 Stunden, bevorzugter ungefähr 1 Minute bis 1 Stunde verabreicht werden.

7. Acetyl-Leucin und Miglustat zur Verwendung nach einem der Ansprüche 1 bis 6, wobei Acetyl-Leucin und Miglustat oral verabreicht werden.

8. Acetyl-Leucin und Miglustat zur Verwendung nach einem der Ansprüche 1 bis 7, wobei Acetyl-Leucin einmal, zweimal oder dreimal täglich verabreicht wird.

9. Acetyl-Leucin und Miglustat zur Verwendung nach einem der Ansprüche 1 bis 8, wobei Miglustat einmal, zweimal oder dreimal täglich verabreicht wird.

10. Acetyl-Leucin und Miglustat zur Verwendung nach einem der Ansprüche 1 bis 9, wobei ungefähr 3 g bis ungefähr 15 g Acetyl-Leucin täglich verabreicht werden.

11. Acetyl-Leucin und Miglustat zur Verwendung nach einem der Ansprüche 1 bis 10, wobei ungefähr 0,05 g bis ungefähr 2 g Miglustat täglich verabreicht werden.

12. Acetyl-Leucin und Miglustat zur Verwendung nach einem der Ansprüche 1 bis 11, wobei Acetyl-Leucin und Miglustat als Primärbehandlung zur Behandlung der lysosomalen Speicherkrankheit verabreicht werden.

13. Acetyl-Leucin und Miglustat zur Verwendung nach einem der Ansprüche 1 bis 12, wobei die lysosomale Speicherkrankheit eine Niemann-Pick-Krankheit, Typ C, ist.

14. Acetyl-Leucin und Miglustat zur Verwendung nach einem der Ansprüche 1 bis 13, wobei das Acetyl-Leucin N-Acetyl-DL-Leucin oder N-Acetyl-L-Leucin ist.

15. Kit, umfassend Acetyl-Leucin und Miglustat zur Verwendung in einem Verfahren zur Behandlung einer lysosomalen Speicherkrankheit bei einem Patienten, wobei der Patient bisher noch nicht mit Miglustat behandelt wurde, wobei das Kit optional ferner Anweisungen zum Verabreichen von Acetyl-Leucin und Miglustat an den Patienten umfasst.

## Revendications

1. Acétyl-leucine et miglustat pour une utilisation dans un procédé de traitement d'une maladie lysosomale chez un sujet en ayant besoin, le procédé comprenant l'administration d'une combinaison :
(i) d'une quantité thérapeutiquement efficace d'acétyl-leucine ; et
(ii) d'une quantité thérapeutiquement efficace de miglustat,
au sujet, le sujet étant naïf au traitement avec le miglustat.

2. Acétyl-leucine et miglustat pour une utilisation selon la revendication 1, l'acétyl-leucine et le miglustat étant administrés simultanément.

3. Acétyl-leucine et miglustat pour une utilisation selon la revendication 2, l'acétyl-leucine et le miglustat étant administrés en tant que formulation pharmaceutique unique ou en tant que deux formulations pharmaceutiques séparées.

4. Acétyl-leucine et miglustat pour une utilisation selon la revendication 1, l'acétyl-leucine et le miglustat étant administrés séquentiellement.

5. Acétyl-leucine et miglustat pour une utilisation selon la revendication 4, l'acétyl-leucine étant administrée avant ou après le miglustat.

6. Acétyl-leucine et miglustat pour une utilisation selon la revendication 5, l'acétyl-leucine et le miglustat étant administrés à part environ 1 minute à environ 6 heures, de préférence l'acétyl-leucine et le miglustat étant administrés à part environ 1 minute à 3 heures, plus préférentiellement à part environ 1 minute à 1 heure.

7. Acétyl-leucine et miglustat pour une utilisation selon l'une quelconque des revendications 1 à 6, l'acétyl-leucine et le miglustat étant administrés oralement.

8. Acétyl-leucine et miglustat pour une utilisation selon l'une quelconque des revendications 1 à 7, l'acétyl-leucine étant administrée une fois, deux fois ou par jour.

9. Acétyl-leucine et miglustat pour une utilisation selon l'une quelconque des revendications 1 à 8, le miglustat étant administré une fois, deux fois ou trois fois par jour.

10. Acétyl-leucine et miglustat pour une utilisation selon l'une quelconque des revendications 1 à 9, environ 3 g à environ 15 g d'acétyl-leucine étant administrés par jour.

11. Acétyl-leucine et miglustat pour une utilisation selon l'une quelconque des revendications 1 à 10, environ 0,05 g à environ 2 g de miglustat étant administrés par jour.

12. Acétyl-leucine et miglustat pour une utilisation selon l'une quelconque des revendications 1 à 11, l'acétyl-leucine et le miglustat étant administrés en tant que thérapie de première intention pour traiter la maladie lysosomale.

13. Acétyl-leucine et miglustat pour une utilisation selon l'une quelconque des revendications 1 à 12, la maladie lysosomale étant une maladie de Niemann-Pick de type C.

14. Acétyl-leucine et miglustat pour une utilisation selon l'une quelconque des revendications 1à 13, l'acétyl-leucine étant la N-acétyl-DL-leucine ou la **N-**acétyl-L-leucine.

15. Ensemble comprenant de l'acétyl-leucine et du miglustat pour une utilisation dans un procédé de traitement d'une maladie lysosomale chez un sujet, dans lequel le sujet est naïf au traitement avec le miglustat, éventuellement, ledit ensemble comprenant en outre des instructions pour l'administration de l'acétyl-leucine et du miglustat au sujet.
